(19) Europäisches Patentamt
European Patent Office
Office européen des brevets

(11) EP 0 650 599 B1

(12) FASCICULE DE BREVET EUROPEEN

(45) Date de publication et mention de la délivrance du brevet: **03.11.1999 Bulletin 1999/44**

(21) Numéro de dépôt: **94916258.0**

(22) Date de dépôt: **06.05.1994**

(51) Int Cl.6: **G01N 33/62**, C07D 209/04

(86) Numéro de dépôt international: **PCT/FR94/00530**

(87) Numéro de publication internationale: **WO 94/27155 (24.11.1994 Gazette 1994/26)**

# (54) PROCEDE DE DOSAGE COLORIMETRIQUE DU DIALDEHYDE MALONIQUE ET DES 4-HYDROXY-2-ENALDEHYDES EN TANT QU'INDICES DE PEROXYDATION LIPIDIQUE, NECESSAIRES POUR SA MISE EN UVRE, INDOLES SUBSTITUES UTILISABLES DANS CE PROCEDE ET LEUR PREPARATION

VERFAHREN ZUR KOLORIMETRISCHEN BESTIMMUNG VON MALONDIALDEHYD UND VON 4-HYDROXY-2-ENALDEHYDEN ALS ANZEIGER DER LIPIDPEROXIDATION, MITTEL ZU SEINES DURCHFÜHRUNG, SUBSTITUIERTE INDOLE ZUR VERWENDUNG IN DIESEM VERFAHREN UND DEREN HERSTELLUNG

METHOD OF COLORIMETRIC ANALYSIS OF MALONIC DIALDEHYDE AND 4-HYDROXY-2-ENALDEHYDES AS INDEXES OF LIPID PEROXIDATION, KITS FOR CARRYING OUT SAID METHOD, SUBSTITUTED INDOLES FOR USE IN SAID METHOD AND THEIR PREPARATION

(84) Etats contractants désignés:
**CH DE ES FR GB IT LI NL SE**

(30) Priorité: **06.05.1993 FR 9305430**

(43) Date de publication de la demande:
**03.05.1995 Bulletin 1995/18**

(73) Titulaire: **OXIS Isle of Man, Limited**
**Douglas 1M1 3DB, Isle of Man (GB)**

(72) Inventeurs:
- **GERARD MONNIER, Dominique**
  **F-94400 Vitry-sur-Seine (FR)**
- **ERDELMEIER, Irène**
  **F-75013 Paris (FR)**
- **CHAUDIERE, Jean**
  **F-94100 Saint-Maur-des-Fossés (FR)**
- **YADAN, Jean-Claude, Yomtob**
  **F-75011 Paris (FR)**

(74) Mandataire: **Portal, Gérard et al**
**Cabinet Beau de Loménie**
**158, rue de l'Université**
**75340 Paris Cédex 07 (FR)**

(56) Documents cités:
**US-A- 4 522 808**

- **FREE RADICAL BIOLOGY & MEDICINE, vol.9, no.6, Juin 1990, NEW YORK, NY, US pages 515 - 540 DAVID R. JANERO 'Malondialdehyde and Thiobarbituric Acid-Reactivity as Diagnostic Indices of Lipid Peroxidation and Peroxidative Tissue Injury' cité dans la demande**
- **METHODS IN ENZYMOLOGY, vol.186, 1990, SAN DIEGO, CA, US pages 407 - 421 HERMANN ESTERBAUER AND KEVIN H.CHEESEMAN 'Determination of Aldehydic Lipid Peroxidation Products: Malonaldehyde and 4-Hydroxynonenal' cité dans la demande**
- **METHODS IN ENZYMOLOGY, vol.186, 1990, SAN DIEGO, CA, US pages 421 - 431 H. H. DRAPER AND M. HADLEY 'Malondialdehyde Determination as Index of Lipid Peroxidation' cité dans la demande**
- **JOURNAL OF THE CHEMICAL SOCIETY(C), vol.1971, 1971, LETCHWORTH GB pages 2606 - 2609 N. P. BUU-HO ET AL. 'Carcinogenic Nitrogen Compounds. Part LXXII. The Möhlau-Bischler Reaction as a Preparative Route to 2-Arylindoles'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

(Cont. page suivante)

- CHEMICAL ABSTRACTS, vol. 74, no. 23, 7 Juin 1971, Columbus, Ohio, US; abstract no. 125326, BETKERUR, SURESH N. ET AL. 'Synthesis of 1-substituted 5--methoxy-2-phenyl-indole derivatives' page 444 ; & JOURNAL OF THE KARNATAK UNIVERSITY, vol.13, 1968, DHARWAR, INDIA pages 32 - 37

## Description

L'invention a pour objets un procédé de dosage colorimétrique du dialdéhyde malonique (1,3-propanedial ou MDA) et des 4-hydroxy-2-énaldéhydes qui se forment au cours du processus de peroxydation lipidique, au moyen de composés de type indoles substitués, des nécessaires ou kits pour la mise en oeuvre de ce procédé, de nouveaux dérivés de l'indole utilisables en tant que réactifs dans la mise en oeuvre de ce procédé et leur préparation.

Dans l'état de la technique antérieure de la préparation des indoles substitués, il est connu par le document US-A-4,522,808 des compositions destinées à lutter contre les brulures solaires contenant des dérivés 2-phénylindole pouvant présenter une multitude de significations, notamment par le fait que sur le cycle indole, il peut y avoir jusqu'à 6 substituants $R^1$ à $R^7$. En outre, sur la partie benzènique de l'indole, les substituants $R^4$ à $R^7$ peuvent avoir diverses significations et en particulier représenter un radical alcoxyle de 1 à 4 atomes de carbone ou un radical carboxyalkyle qui en réalité est un groupe de formule alkylène carboxylique $(CH_2)_nCOOH$.

Dans le cadre de la présente invention, on peut utiliser des nouveaux dérivés 2-arylindoles comme par exemple les dérivés 2-phénylindoles qui sont cependant différents de ceux décrits dans ce document antérieur en particulier en ce que les substituants de la partie phényle du noyau indole ne peuvent pas représenter un radical alcoxyle ni un radical alkyle ou alkylène carboxylique.

D'autre part, le document J. Chem. Soc. (C 1971, pages 2606-2608) décrit des dérivés 2-arylindoles, en particulier des dérivés indoles diméthylés ou triméthylés, composés qui sont différents de ceux objets de la présente invention.

Enfin, le document Chemical Abstracts, volume 74, 971, pages 444, abrégé 125326v, décrit des composés pour lesquels $R^2 = OCH_3$ et $R^3$ = méthyle, composés qui sont différents de ceux objets de la présente invention.

Dans certains états pathologiques tels que par exemple la pré-éclampsie, l'infarctus du myocarde et les états de choc, l'oxydation des lipoprotéines circulantes ou de certaines membranes cellulaires conduit à la formation de peroxydes lipidiques qui se décomposent. Les états pathologiques concernés peuvent être mis en évidence, ou leur évolution peut être suivie, grâce au dosage des produits de décomposition de ces acides gras insaturés peroxydés.

L'expression "peroxydation lipidique", telle qu'utilisée ici, vise l'ensemble des voies réactionnelles qui conduisent à la formation d'hydroperoxydes, de dialkyl-peroxydes et d'endoperoxydes d'acides gras polyinsaturés (AGPI) ou de leurs esters (phospholipides, cholestéryl-esters, mono-, di- et triglycérides en particulier), ainsi qu'à leur décomposition.

La formation de "peroxydes lipidiques" peut être d'origine non-enzymatique: oxydation spontanée en présence d'oxygène à l'état fondamental, addition d'oxygène singulet, oxydation catalysée par des complexes de métaux de transition ou par des métalloprotéines partiellement dégradées telles que, par exemple, la ferryl-hémoglobine, la ferryl-myoglobine ou l'hématéine, en présence ou en absence d'hydroperoxydes formés au préalable.

Cette formation, peut également être d'origine enzymatique, c'est-à-dire résulter de l'action d'enzymes telles que des lipoxygénases végétales ou animales, des cyclo-oxygénases ou des oxygénases microsomales à cytochrome P-450.

Elle peut enfin résulter du couplage de réactions enzymatiques et non-enzymatiques. C'est par exemple le cas des oxydations lipidiques associées à la réduction monovalente de certains xénobiotiques, en présence d'oxygène et de systèmes réducteurs enzymatiques.

La décomposition des peroxydes lipidiques aboutit à la formation de très nombreux sous-produits organiques.

Cette décomposition peut être d'origine enzymatique (action de peroxydases et/ou de la thromboxane synthétase, par exemple) ou non-enzymatique (thermique, photochimique, réductrice, ou catalysée par des complexes de métaux de transition).

Parmi les principaux sous-produits organiques de la décomposition des peroxydes lipidiques, on peut citer certains alcools, diènes conjugués, triènes conjugués, hydrocarbures volatils tels que l'éthane, le pentane ou l'éthylène, certains aldéhydes ou cétones saturés, insaturés et/ou hydroxylés, certains époxydes saturés ou insaturés, des bases de Schiff issues de la réaction des aldéhydes précités avec les groupements amine des molécules présentes dans le milieu et les combinaisons de certains composés précités avec des structures moléculaires polyfonctionnelles.

Ainsi définie, l'expression "peroxydation lipidique" s'applique exclusivement aux voies de formation et de dégradation des peroxydes d'AGPI, libres ou estérifiés.

L'oxydabilité marquée des AGPI est due à la présence systématique dans ces composés du motif cis,cis-1,4-pentadiène qui permet une délocalisation stabilisante des intermédiaires radicalaires initialement produits par scission homolytique d'une liaison méthylénique C-H, comme montré sur le schéma qui suit.

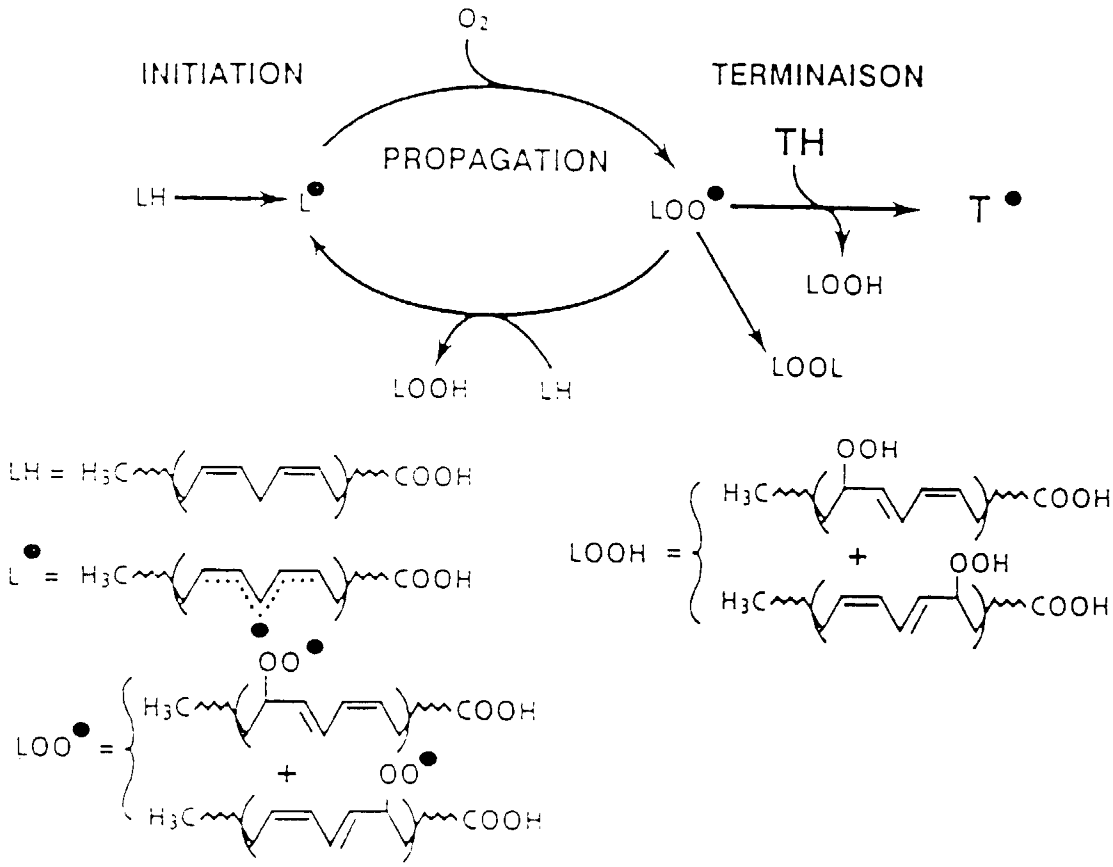


Dans les milieux biologiques, les AGPI les plus importants sont ceux qui sont dérivés de l'acide linoléique et de l'acide linolénique, c'est-à-dire ceux qui appartiennent aux familles dites en "n-6" et en "n-3", respectivement.

Toute mesure d'indice(s) de peroxydation lipidique doit donc être aussi spécifique que possible des produits ou sous-produits de peroxydation des AGPI précités.

Un grand nombre de méthodes de mesure de la peroxydation lipidique ont été décrites dans le passé. On peut les diviser en cinq grandes familles :

1. Méthodes spectrophotométriques directes ou indirectes.
2. Méthodes spectrofluorimétriques.
3. Méthodes électrochimiques.
4. Méthodes chimiluminescentes.
5. Méthodes chromatographiques.

Les méthodes appartenant aux familles 2. à 5. ont été décrites récemment (références 1 et 2). Elles posent des problèmes de spécificité ou de complexité instrumentale qui excluent leur utilisation en routine par des laboratoires non spécialisés.

Les méthodes spectrophotométriques directes connues de la famille 1. dans laquelle s'inscrit la présente invention, sont résumées ci-après.

La méthode classique des "diènes conjugués" est basée sur une mesure d'absorbance à 233 nm qui permet de quantifier les structures bis-allyliques (trans-diéniques) des hydroperoxydes et alcools d'AGPI, avec une sensibilité élevée.

Dans la plupart des échantillons biologiques, la présence d'autres structures diéniques ainsi que l'absorption très intense des groupements carbonyle et des mono-alcènes produisent malheureusement des interférences majeures qui ne peuvent être éliminées que par des procédures de spectroscopie différentielle, généralement incompatibles avec un dosage sur échantillon biologique de composition inconnue.

Le dosage colorimétrique du dialdéhyde malonique (1,3-propanedial ou MDA) fournit l'indice de peroxydation lipidique le plus utilisé (référence 2).

Le MDA est à la fois un métabolite enzymatique de l'acide arachidonique et un produit secondaire de la peroxydation des AGPI (référence 3).

La principale voie de production enzymatique du MDA est celle de la thromboxane synthétase (référence 4) qui convertit la prostaglandine PGH2 (endoperoxyde) en thromboxane A2, 12(L)-hydroxy-5,8,11-heptatriènoate et MDA (dans les proportions respectives 1/3, 1/3, 1/3).

Le MDA d'origine non-enzymatique se forme par décomposition radicalaire d'endoperoxydes mono- ou bicycliques dérivés d'un radical peroxyle bis-allylique.

Les propriétés chimiques et biochimiques du MDA ont fait l'objet d'une revue détaillée (référence 5).

C'est un composé volatil qui est totalement énolisé, sous forme de monoénol-monoaldéhyde, en solution aqueuse ou organique. Il présente à la fois des propriétés nucléophiles (énolate) et électrophiles (aldéhyde), ce qui explique sa tendance à se polymériser pour donner un mélange d'adduits multimériques plus ou moins réversibles.

Lorsqu'il est chauffé à pH acide, le MDA réagit avec un certain nombre de composés nucléophiles pour donner divers produits de condensation.

Des méthodes de quantification ou de dosage de certains des produits de condensation du MDA ont été proposées mais aucune n'est totalement satisfaisante.

Le pyrazol-benzothiazole dérivé du 2-hydrazino-benzothiazole, ou le 1-méthylpyrazole dérivé de la méthylhydrazine, peuvent être quantifiés par chromatographie en phase gazeuse (méthodes de la famille 5) du fait de leur volatilité élevée.

Le N-méthylpyrrole (référence 6) fournit un adduit chromophorique caractéristique du MDA. Toutefois, sa demi-vie est trop courte pour permettre des mesures colorimétriques sur de grandes séries d'échantillons.

Le 2-méthylindole conduit aussi à un adduit chromophorique avec le MDA, mais le coefficient d'extinction molaire ($\varepsilon_m \approx 32000$ 1.mole$^{-1}$.cm$^{-1}$) est beaucoup trop faible pour permettre un dosage de MDA à des concentrations physiologiques (0,5-5 µmolaires) (référence 7).

En pratique, c'est l'acide thiobarbiturique (TBA) qui est actuellement presque exclusivement utilisé pour doser le MDA par colorimétrie, en raison de la stabilité et du coefficient d'extinction molaire à 530-535 nm élevés du produit de condensation chromophorique qui se forme par addition de deux molécules de TBA sur une molécule de MDA. De plus, ce produit étant fluorescent, il peut également être dosé par spectrofluorométrie.

Toutes les procédures de dosage du MDA et/ou des produits de peroxydation lipidique réagissant avec le TBA, appelés ci-après "TBARS" (TBA-Reactive Substances) requièrent un pH acide (< 3,8) et un chauffage prolongé (80-95°C, 20-60 min). L'acidification et le chauffage des échantillons conduisent généralement à une suspension trouble qui doit être clarifiée avant la mesure, par exemple par extraction au n-butanol.

Les variantes du test au TBA sont très nombreuses, ce qui souligne que cette approche pose des problèmes, notamment en ce qui concerne sa fiabilité (référence 8). En effet, le test au TBA pose deux problèmes majeurs, à savoir :

- les conditions de température et de pH requises provoquent des réactions secondaires conduisant à des produits artéfactuels qui réagissent avec le TBA, faussant ainsi le dosage; et
- le TBA n'est pas un réactif spécifique du MDA.

Une première source d'interférences est due à la présence d'aldéhydes d'origine lipidique, mais distincts du MDA. Un certain nombre de monoaldéhydes réagit avec le TBA dans les conditions classiques du test pour fournir des produits qui interfèrent avec l'absorption de l'adduit MDA:TBA (1:2) (référence 9).

Le rendement de formation de certains pigments est aléatoire, car il dépend des concentrations respectives d'aldéhydes et de certains adduits intermédiaires présents simultanément dans l'échantillon. Certains de ces mécanismes chimiques ont été discutés (référence 10).

Les produits colorés dus à la présence de monoaldéhydes d'origine lipidique ont généralement un ou plusieurs pics d'absorption entre 450 nm et 500 nm qui sont étalés et en recouvrement avec celui de l'adduit MDA:TBA (1:2). Ils peuvent multiplier l'absorption à 530-535 nm par un facteur important car sur un extrait de lipides oxydés, la somme des quantités de monoaldéhydes produits peut excéder la production de MDA (référence 5).

La seconde source d'interférences est due à la formation de MDA ou de TBARS à partir de composés non lipidiques. Le désoxyribose et ses dérivés nucléosidiques (référence 11), de même que la pyrimidine et la 2-aminopyrimidine se comportent comme du MDA authentique dans les conditions du test (référence 8).

Par ailleurs, les sucres tels que le sucrose et le glucose ont un puissant effet de synergie sur la formation de TBARS en présence de peroxydes ou d'aldéhydes divers tels que l'acétaldéhyde (référence 12), alors que ces molécules ne forment pas de TBARS lorsqu'elles sont présentes séparément.

L'acide sialique (acide N-acétylneuraminique) produit un adduit absorbant intensément vers 550 nm (référence 8).

La bilirubine, la biliverdine et le sérum hémolysé produisent également une absorption "contaminante".

Le test au TBA pose donc des problèmes de spécificité dus à :

- la formation de TBARS d'origine lipidique, s'ajoutant au MDA,
- l'existence d'interférences réactionnelles (formation de MDA ou de TBARS) dues à des contaminants d'origine non lipidique et actifs dans les conditions du test.

Le recouvrement des spectres d'absorption ou de fluorescence des adduits contaminants avec le spectre de l'adduit MDA:TBA (1:2) ne permet pas d'améliorer la spécificité des mesures en utilisant de multiples longueurs d'onde.
La séparation chromatographique de l'adduit du MDA ne permet pas non plus d'éliminer les problèmes de formation artéfactuelle de MDA authentique dans les conditions du test au TBA.
Ces difficultés ont conduit un certain nombre de laboratoires à développer des méthodes chromatographiques de dosage du MDA ainsi que d'autres aldéhydes (référence 1).
Ces procédures sont longues et complexes. Elles sont donc difficilement utilisables en routine sur de grandes séries d'échantillons, mais leur développement a permis de préciser l'importance des productions de MDA et d'autres aldéhydes dans les phénomènes de peroxydation lipidique.
Esterbauer et ses collaborateurs ont ainsi montré que la production de 4-hydroxynonénal (4-HNE) était caractéristique des phénomènes de peroxydation lipidique (référence 13).
L'oxydation des AGPI en "n-6" est à l'origine de cette production de 4-HNE, dont le rendement est maximal avec l'acide arachidonique 20:4(n-6).
Un composé voisin, le 4-hydroxyhexénal, est produit à partir des AGPI en "n-3".
En résumé, on sait que le MDA et/ou d'autres énaldéhydes dans un milieu, notamment biologique, constituent des indices de peroxydation lipidique mais on ne dispose pas jusqu'à présent de méthodes fiables et de mise en oeuvre simple pour les doser.
La présente invention a donc notamment pour buts de :

- disposer de nouveaux réactifs réagissant avec les 4-hydroxy-2-énaldéhydes, et le MDA;
- disposer d'un dosage colorimétrique des énaldéhydes et de leurs dérivés acido-labiles tels que acétals, thioacétals, imines, éthers d'énols ou thioéthers d'énols en tant qu'indices de peroxydation lipidique, par production quantitative de produits de condensation stables et porteurs d'une même structure chromophorique unique dont la longueur d'onde d'absorption maximale soit supérieure ou égale à 570 nm, étant entendu que le terme "énaldéhyde" s'applique à l'ensemble comprenant les 4-hydroxy-2-énaldéhydes et le MDA. Les 4-hydroxy-2-énaldéhydes peuvent être par exemple le 4-hydroxynonénal, le 4-hydroxyhexénal, le 4-hydroxydécénal et le 4-hydroxydodécénal;
- permettre un dosage colorimétrique spécifique du MDA à la même longueur d'onde d'absorption que celle du dosage des 4-hydroxy-2-énaldéhydes.

Ces buts sont atteints grâce à l'invention qui utilise des réactifs chimiques originaux et qui exploite les propriétés chromogènes des produits d'addition de ces réactifs aux énaldéhydes, tels que définis précédemment.
Ces réactifs sont caractérisés, en particulier, par la présence d'un noyau indolique ou benz(f)indolique non substitué en position 3 et par leur solubilité dans des solvants protiques polaires tels que l'eau, les alcools méthylique et éthylique, l'acétonitrile, entre autres. Le chromophore généré par addition de ces réactifs aux énaldéhydes, tels que définis précédemment, présente une longueur d'onde d'absorption maximale qui est toujours supérieure ou égale à 570 nm.
L'invention fournit un procédé simple et rapide mettant en oeuvre ces réactifs, procédé qui permet de doser par colorimétrie des énaldéhydes, tels que définis précédemment, et leurs dérivés acido-labiles tels que acétals, thioacétals, imines, éthers d'énols ou thioéthers d'énols en tant qu'indices de peroxydation lipidique, par production quantitative de produits de condensation stables et porteurs d'une même structure chromophorique unique, dans des conditions de température beaucoup plus douces que la méthode au TBA, ce qui limite considérablement les artéfacts potentiels dus à un chauffage à haute température pendant une longue durée. Ce procédé est donc bien adapté à un dosage de routine sur de grandes séries d'échantillons.
Plus précisément, selon l'un de ses aspects, l'invention a pour objet un procédé de dosage colorimétrique des énaldéhydes, tels que définis précédemment, ou du dialdéhyde malonique seul, en tant qu'indices de peroxydation lipidique dans un milieu, notamment biologique, caractérisé en ce qu'il comprend essentiellement :

a) l'addition au dit milieu d'un réactif choisi parmi les composés de formule générale I ci-dessous et leurs sels d'addition éventuels à des bases organiques ou inorganiques, ou à des acides organiques ou inorganiques :

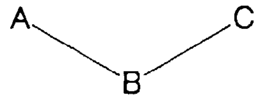

(I)

formule dans laquelle :

- A et C qui peuvent être identiques ou différents, représentent chacun H,

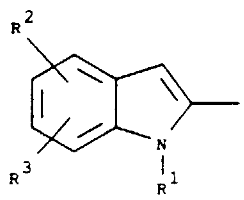

ou

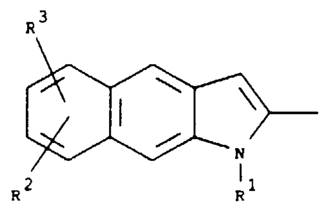

étant entendu que A et C ne peuvent représenter simultanément H, avec :

- $R^1$ = H; alkyle; aralkyle; aralkyle substitué sur le noyau aryle; alkylsulfonate, $Y^+$; alkylphosphonate, $Y^+$; ou alkylcarboxylate, $Y^+$;
- $R^2$ = H; $-OR^4$; F; Cl; Br; I; $-NO_2$; $-SO_3^-$ $Y^+$; -CN; $-COOR^4$; ou $-CONR^5R^6$;
- $R^3$ = H; $-OR^4$; $-NR^5R^6$; $-SR^4$; F; Cl; Br; I; $-NO_2$; $-SO_3^-$ $Y^+$;
    - CN; $-COR^5$; $-COOR^4$; ou $-CONR^5R^6$;
    - $R^4$ = H; alkyle; aralkyle; ou aralkyle substitué sur le noyau aryle;
    - $R^5$ = H; alkyle; aralkyle; ou aralkyle substitué sur le noyau aryle;
    - $R^6$ = H; aryle; aralkyle; ou aralkyle substitué sur le noyau aryle;
    - $Y^+$ = cation d'une base organique ou inorganique;
- B =

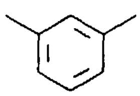 , 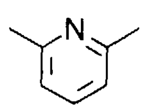 , 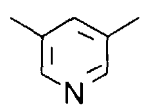 , 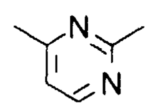 ,

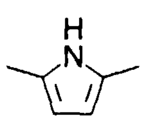 , 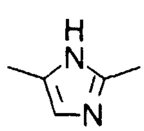 , 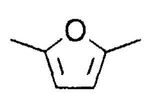 ,

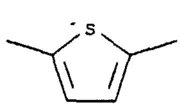 ou 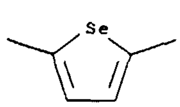

étant entendu que :

- alkyle représente un groupement linéaire ou ramifié comprenant 1 à 6 C;
- aralkyle substitué sur le noyau aryle signifie que celui-ci est substitué par un ou plusieurs groupements, identiques ou différents, choisis parmi alkyle (1 à 6 C), alcoxy (1 à 6 C), hydroxy, amino et carboxyle;

b) l'acidification du milieu ainsi additionné, soit par un acide choisi dans le groupe constitué par les acides carboxyliques forts, les acides sulfoniques forts et l'acide perchlorique, soit par l'acide chlorhydrique, l'acide bromhydrique ou l'acide sulfurique;
c) l'incubation du milieu ainsi acidifié à une température comprise entre 25 et 60°C, pendant une durée suffisante pour obtenir une coloration stable due à la formation d'un produit d'addition chromophorique du réactif avec tous les énaldéhydes, tels que définis précédemment, présents dans le milieu ou avec le dialdéhyde malonique seul, selon la nature de l'acide utilisé;
d) la mesure de l'absorbance du milieu coloré à la longueur d'onde d'absorption qui est spécifique du produit d'addition chromophorique du réactif utilisé, et son utilisation pour déterminer la concentration en énaldéhydes, tels que définis précédemment, ou en dialdéhyde malonique seul, selon le cas.

Lorsque dans le composé de formule générale I choisi, $R^4$ = H, on peut utiliser son sel d'addition à une base inorganique ou organique qui peut être choisie par exemple parmi l'hydroxyde de sodium, l'hydroxyde de potassium, les carbonates de métaux alcalins, les carbonates de métaux alcalino-terreux, la triéthylamine, l'arginine.

Lorsque, dans le composé de formule générale I choisi, $R^5$ et/ou $R^6$ = H, on peut utiliser son sel d'addition à un acide inorganique ou organique qui peut être choisi par exemple parmi les acides chlorhydrique, bromhydrique, iodhydrique, sulfurique, tartrique, méthanesulfonique, trifluorométhanesulfonique.

L'incubation conduisant à la formation du chromophore doit avoir lieu en milieu acide.

La durée d'incubation dépend essentiellement de la température utilisée. Elle ne dépasse en général pas 1 heure et lui est le plus souvent inférieure.

Selon l'invention, on a trouvé que les réactifs de formule générale I réagissent, en présence de certains acides, avec tous les énaldéhydes, tels que définis précédemment, résultant du processus de peroxydation lipidique tel que défini ci-dessus ou le MDA seul, pour conduire à un produit d'addition chromophorique stable sur une longue période, dont la longueur d'onde d'absorption maximale est dans tous les cas supérieure ou égale à 570 nm et dépend de la nature du réactif de formule I.

Plus précisément, en présence d'un acide carboxylique fort, d'un acide sulfonique fort ou de l'acide perchlorique, les réactifs de formule générale I réagissent avec tous les énaldéhydes, tels que définis précédemment, présents dans le milieu, alors qu'en présence d'acide chlorhydrique, d'acide bromhydrique ou d'acide sulfurique, ils ne réagissent pratiquement qu'avec le MDA, pour former le composé chromophorique caractéristique du réactif utilisé.

Chaque acide est avantageusement conditionné séparément. Il doit être de qualité ultra-pure et est avantageusement conservé sous forme pure ou de solution-mère dilué dans l'eau désionisée, à une concentration non inférieure à 5 moles par litre.

Dans le cadre du procédé, il est avantageux que la concentration finale de l'acide dans le milieu réactionnel final soit généralement comprise entre 0,5 et 2,5 moles par litre.

En ce qui concerne le réactif de formule générale (I) précitée, celui-ci peut se présenter avantageusement sous forme d'une solution-mère dans un solvant organique compatible comme par exemple un solvant organique miscible à l'eau tel que notamment l'acétonitrile, le DMSO, le THF, le méthanol, l'éthanol ou l'isopropanol, ou bien dans l'eau neutre ou acidifié ou dans un tampon.

La concentration du réactif dans sa solution-mère est en général avantageusement comprise entre 5 et 40 mmoles par litre, selon sa solubilité dans le solvant utilisé.

Ce réactif sera le plus souvent dilué au moment de l'emploi pour obtenir une concentration finale dans le milieu réactionnel final avantageusement comprise entre 3 et 25 mmoles/litre.

L'invention permet donc de doser non seulement tous les énaldéhydes, tels que définis précédemment, présents dans le milieu testé, mais aussi de doser spécifiquement le MDA en présence de 4-hydroxy-2-énaldéhydes, dans un échantillon, notamment biologique.

Le procédé selon l'invention permet donc de mesurer la quantité globale d'énaldéhydes, tels que définis précédemment, sur une première prise d'échantillon, puis celle de MDA sur une seconde prise d'échantillon, ou le contraire. Les conditions opératoires très douces de ce nouveau procédé, en permettant d'éliminer la plupart des artéfacts très nombreux liés à la méthode au TBA, en font une méthode particulièrement fiable et reproductible.

L'ensemble des réactifs de formule générale I conduisent à la formation d'un chromophore dont la structure commune est du type :

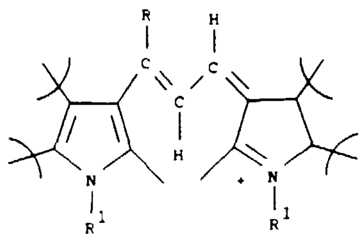

Dans le cas du MDA, la formation du chromophore ne requiert aucune étape d'oxydation, tandis que dans le cas des 4-hydroxy-2-énaldéhydes, une oxydation est nécessaire et le milieu réactionnel ne peut donc être dégazé en oxygène ambiant.
En tant qu'acide carboxylique fort qui peut être utilisé, on peut citer l'acide trifluoroacétique.
En tant qu'acides sulfoniques forts qui peuvent être utilisés, on peut citer l'acide méthanesulfonique et l'acide trifluorométhanesulfonique.
On a trouvé cependant que selon le degré de pureté des acides et réactifs utilisés, le rendement de formation du chromophore pouvait varier de façon significative lorsque d'autres énaldéhydes que le MDA sont impliqués dans la réaction.
Selon un autre aspect de l'invention, on a trouvé que l'addition d'un sel métallique tel que par exemple un sel de fer ferrique (5 micromolaire) dans le milieu réactionnel final permettait d'obtenir un rendement reproductible et maximal de formation du chromophore avec tous les 4-hydroxy-2-énaldéhydes testés.
La nouvelle méthode de dosage selon l'invention constitue un nouvel outil pour les recherches biologiques en général ainsi qu'en Chimie clinique, en particulier pour l'élaboration de kits de dosage utilisables en recherche ou en contrôle de qualité, ou bien encore dans le domaine du diagnostic médical.
La méthode peut ainsi être utilisée pour évaluer le degré de rancidité des produits alimentaires ainsi que celui des solutions nutritives utilisées par voie entérale ou parentérale.
Dans le domaine du diagnostic médical, la méthode peut être utilisée pour confirmer un état pathologique tel que la pré-éclampsie chez la femme enceinte et hypertendue, l'angor instable, l'ischémie myocardique silencieuse.
Elle peut également être utilisée pour valider l'efficacité d'une intervention de thrombolyse artérielle ou d'angioplastie.
Elle peut enfin fournir un indice d'efficacité thérapeutique lors du traitement des états de choc (septique ou traumatique), de certaines intoxications aigües, du diabète, du SIDA, de certains états de malnutrition, et des maladies génétiques de surcharge en fer.
Elle peut enfin être utilisée dans le suivi post-opératoire des transplantations d'organes et/ou des procédures de circulation extra-corporelle.
Les échantillons visés sont notamment des échantillons biologiques tels que le plasma sanguin entier ou déprotéinisé, les lysats de globules rouges, le liquide céphalo-rachidien, les lipoprotéines plasmatiques obtenues par précipitation sélective ou les homogénats de tissus solides.
L'échantillon à doser peut être essentiellement liquide comme par exemple un extrait, une solution ou une dispersion d'un matériel, notamment biologique, dans un milieu aqueux éventuellement tamponné ou dans un solvant organique miscible à l'eau.
Il peut s'agir par exemple du culot de centrifugation d'un échantillon biologique dispersé en milieu aqueux, éventuellement tamponné, ou organique.
Les échantillons liquides peuvent être des solutions aqueuses déprotéinisées par précipitation au moyen d'acides tels que par exemple l'acide métaphosphorique, l'acide trichloroacétique ou l'acide phosphotungstique.
Ces échantillons peuvent contenir un détergent neutre ou cationique (à raison notamment de 0 à 2 g par 100 ml), par exemple du Lubrol(r) (condensat d'oxyde de polyéthylène) ou un sel de tétra-alkylammonium.
En tout état de cause, la proportion volumique de l'échantillon dans le milieu réactionnel final (après addition de l'acide) doit être compatible avec l'obtention d'une seule phase liquide dans ce milieu.
L'incubation de l'échantillon avec le réactif de formule I pour former le composé d'addition chromogène doit être effectuée à une température ne dépassant pas 60°C, afin d'éviter des réactions secondaires conduisant à des produits artéfactuels dont la présence fausserait le dosage.
Selon le réactif de formule I utilisé, le temps d'incubation du milieu réactionnel à la température choisie doit être fixé de manière à atteindre un plateau stable correspondant à un rendement maximal de production du chromophore mesuré. Le temps d'incubation est en général inférieur ou égal à 60 minutes.
Pour le dosage des énaldéhydes, tels que définis précédemment, le milieu réactionnel final ne doit pas être dégazé. Il peut l'être lorsque l'on dose le MDA seul, de façon spécifique. Toutefois, d'une manière générale, il est

préférable que le milieu réactionnel final soit constitué de solutions et solvants non préalablement dégazés en oxygène ambiant.

La mesure spectrophotométrique de l'absorbance du chromophore produit par réaction des énaldéhydes, tels que définis précédemment, ou du MDA seul avec un réactif chromogène de formule générale I et en présence d'un acide fort, doit être effectuée à une longueur d'onde fixe et voisine (+ 5 nm) de la longueur d'onde d'absorption maximale (> 570 nm) du chromophore spécifique du réactif utilisé.

Selon un mode avantageux de réalisation, la concentration en énaldéhydes, tels que définis précédemment, ou en dialdéhyde malonique seul, selon le cas, est déterminée au moyen d'une courbe d'étalonnage.

Cette courbe d'étalonnage peut être réalisée à partir d'une solution aqueuse ou organique de concentration connue de l'un quelconque des énaldéhydes, tels que définis précédemment, ou de leurs précurseurs acido-labiles tels que définis précédemment. Le solvant doit être choisi de manière à éviter toute polymérisation de l'aldéhyde utilisé ou toute hydrolyse du précurseur utilisé, pendant la période d'utilisation de la solution.

On utilise avantageusement une solution standard contenant un énaldéhyde susceptible de se trouver dans le milieu à doser, par exemple du MDA ou du 4-hydroxynonénal (4-HNE), ou tout autre 4-hydroxy-2-énaldéhyde habituellement formé au cours du processus de peroxydation lipidique.

La solution standard utilisée doit présenter une concentration telle qu'elle puisse être diluée pour obtenir plusieurs points de la courbe d'étalonnage.

Pour obtenir une courbe d'étalonnage, on porte, en fonction de la concentration connue en énaldéhyde, en travaillant dans les conditions (acidification et incubation) du dosage prévu, la différence de l'absorbance A pour une concentration donnée et de l'absorbance $A_0$ d'un témoin ne contenant pas d'aldéhyde, ces absorbances étant lues à la longueur d'onde d'absorption maximale caractéristique du produit d'addition entre les énaldéhydes, tels que définis précédemment, et le réactif utilisé.

On obtient ainsi une droite dont la pente est égale au coefficient d'extinction molaire apparent E du composé d'addition mesuré.

Différents essais avec notamment le MDA, le 4-hydroxynonénal et le 4-hydroxyhexénal ont montré que quel que soit l'énaldéhyde utilisé, la pente de la droite est pratiquement la même pour un même réactif de formule I donné. On peut donc utiliser, pour établir la courbe d'étalonnage, l'un quelconque des énaldéhydes, tels que définis précédemment, susceptibles d'être présents dans le milieu à doser.

On utilise avantageusement le MDA ou le 4-hydroxynonénal qui sont facilement accessibles.

Dans le cas particulier du dosage du MDA seul, la courbe d'étalonnage est de préférence établie dans les conditions particulières de ce dosage (acidification par l'acide chlorhydrique ou l'acide bromhydrique), en utilisant le MDA qui, comme il sera vu plus en détail dans la partie expérimentale qui suit, est pratiquement le seul énaldéhyde qui réagit pour conduire au chromophore dans ces conditions.

La concentration en énaldéhydes, tels que définis précédemment, recherchés peut être déterminée à partir de la mesure de l'absorbance A de l'échantillon et de la mesure de l'absorbance $A_0$ faite sur un témoin ne contenant pas d'aldéhyde et du coefficient d'extinction molaire E qui correspond à la pente de la droite d'étalonnage.

On aura ainsi :

$$[\text{concentration en énaldéhydes, tels que définis précédemment,}] = (A\text{-}A_0) \times K / E.$$

Dans cette équation :

- la concentration en énaldéhydes tels que définis précédemment, est exprimée en moles.$l^{-1}$;
- A et $A_0$ sont lues avec un trajet optique de 1 cm et sont exprimées en $cm^{-1}$;
- K est le facteur de dilution; et
- E est exprimé en 1-$moles^{-1}$-$cm^{-1}$.

La concentration en énaldéhydes, tels que définis précédemment, peut également se déduire directement de la courbe d'étalonnage à partir de la mesure A, en utilisant la différence A-$A_0$.

Selon un autre de ses aspects, l'invention a pour objet un nécessaire ou kit pour la mise en oeuvre du procédé de dosage décrit ci-dessus, caractérisé en ce qu'il comporte essentiellement un réactif répondant à la formule générale I définie ci-dessus.

Ce réactif se présente avantageusement sous forme d'une solution-mère dans un solvant organique compatible comme par exemple un solvant organique miscible à l'eau tel que notamment l'acétonitrile, le DMSO, le THF, le méthanol, l'éthanol ou l'isopropanol ou bien dans l'eau neutre ou acidifiée ou dans un tampon.

La concentration du réactif dans sa solution-mère est en général comprise entre 5 et 40 mmoles/l, selon sa solubilité dans le solvant utilisé.

Ce réactif sera le plus souvent dilué au moment de l'emploi pour obtenir une concentration finale dans le milieu réactionnel final avantageusement comprise entre 3 et 25 mmoles/l.

Selon un mode préféré de réalisation, le nécessaire ou kit selon l'invention comprend en outre au moins un acide fort choisi dans le groupe constitué par les acides organiques forts comme l'acide trifluoroacétique, les acides sulfoniques forts comme l'acide méthanesulfonique ou l'acide trifluorométhanesulfonique, l'acide perchlorique, l'acide sulfurique, l'acide chlorhydrique et l'acide bromhydrique.

Chaque acide est conditionné séparément. Il doit être de qualité ultra-pure et est avantageusement conservé sous forme pure ou de solution-mère diluée dans l'eau désionisée, à une concentration non inférieure à 5 moles/l.

La concentration finale de l'acide dans le milieu réactionnel final sera en général comprise entre 0,5 et 2,5 moles/l.

Selon un autre mode préféré de réalisation, le nécessaire ou kit selon l'invention comprend en outre, en tant que référence ou échantillon standard, au moins un énaldéhyde, tel que défini précédemment, indice de la peroxydation lipidique ou un précurseur acido-labile d'un tel énaldéhyde, en solution aqueuse ou organique, à une concentration connue.

Les composés de formule générale I peuvent être préparés selon les procédures générales 1 à 3 décrites ci-dessous.

**Procédure générale 1: méthode de Fischer**

Cette procédure générale de préparation des composés de formule générale I est basée sur les méthodes décrites dans la littérature (référence 14).

Elle comporte essentiellement trois étapes à partir d'un chlorhydrate d'arylhydrazine substitué ou non, comme résumé sur le schéma I suivant :

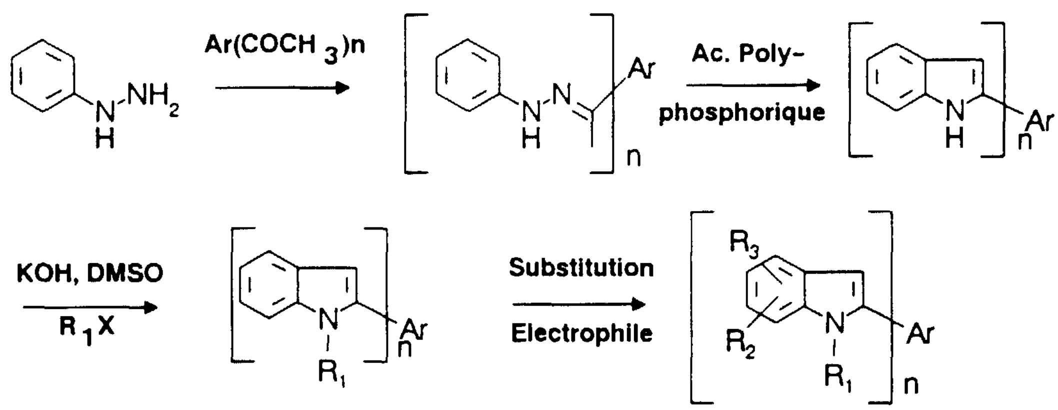


n = 1 ou 2

**- SCHEMA I -**

A une solution de chlorhydrate d'arylhydrazine commercial est ajoutée, au reflux, une solution du dérivé aromatique acétylé correspondant, lui-même commercial ou préparé selon une des méthodes connues (référence 15). Le reflux est maintenu pendant 15-90 minutes. Après refroidissement, le produit désiré précipite ou cristallise. Dans certains cas, une purification par chromatographie liquide sur colonne de silice s'avère nécessaire.

L'étape de cyclisation est réalisée à l'aide d'acide polyphosphorique selon la procédure classique. Le produit désiré est obtenu après purification par chromatographie liquide sur colonne d'alumine et est recristallisé.

L'alkylation du dérivé indolique nécessite une première phase de génération de l'anion par réaction avec de la potasse en poudre dans le diméthylsulfoxyde à température ambiante pendant plusieurs heures; puis l'agent alkylant, comme par exemple le sulfate de méthyle, est ajouté. Après traitement usuel, le produit désiré est obtenu après purification par chromatographie liquide sur colonne de silice et est recristallisé.

Les exemples 1 à 7 illustrent cette procédure générale 1.

**Procédure générale 2 : selon la référence 16**

Cette procédure comporte essentiellement trois étapes à partir d'un composé de type 2-éthynylaniline substitué ou non, comme résumé sur le schéma II suivant :

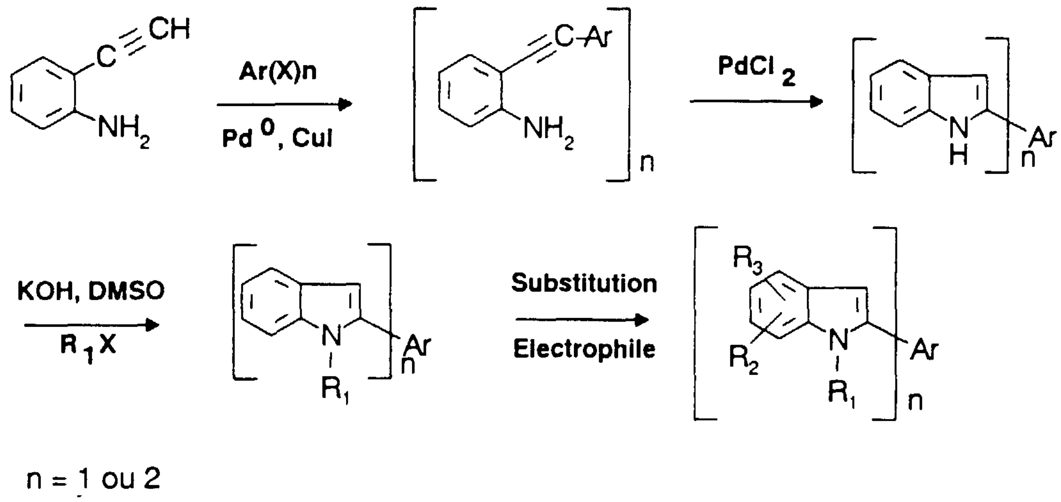


**- SCHEMA II-**

Le composé de type 2-éthynylaniline est soumis à l'action d'un halogénure ou d'un trifluorométhanesulfonate d'aryle en présence de palladium zéro ($Pd^0$) et d'iodure cuivreux (CuI) à température ambiante pendant 1 à 6 heures. Le dérivé aryle substitué correspondant est obtenu après le traitement usuel et purification par chromatographie liquide sur colonne de silice ou d'alumine.

L'étape de cyclisation est réalisée à l'aide d'une quantité catalytique de chlorure de palladium ($PdCl_2$) en chauffant le dérivé précédent dans l'acétonitrile au reflux pendant 2-30 heures. Le produit désiré est obtenu après chromatographie liquide sur colonne de silice ou d'alumine, ou après recristallisation.

L'alkylation est réalisée de la même façon que pour la procédure 1.

Les exemples 8 à 11 illustrent cette procédure générale 2.

**Procédure générale 3 : selon la référence 17**

Cette procédure comporte essentiellement quatre étapes à partir d'un composé de type 2-halogénoaniline ou 3-amino-2-halogénonaphtalène substitué ou non, comme résumé sur le schéma III suivant :

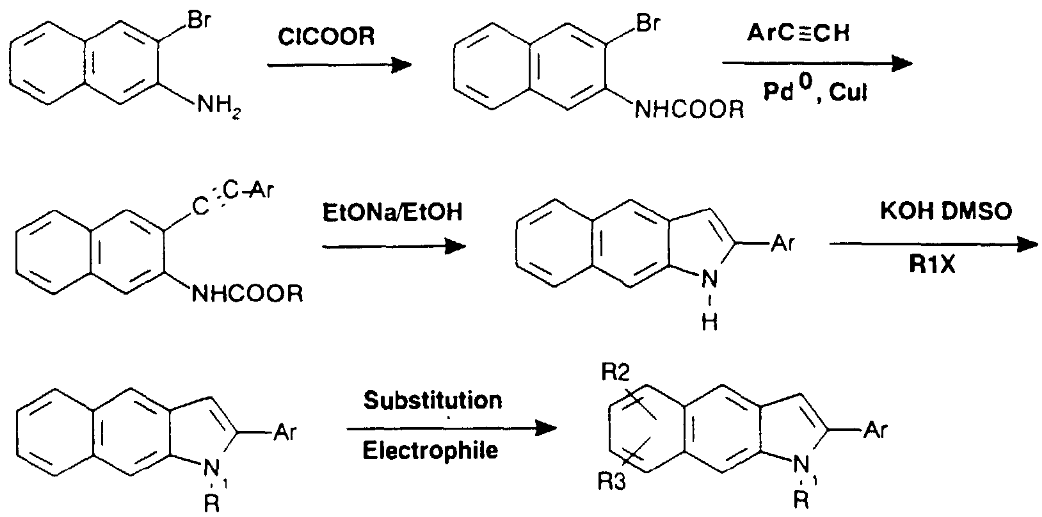


**- SCHEMA III -**

Le composé 2-bromoaniline ou 3-amino-2-bromonaphtalène de départ, selon le cas, est converti en dérivé carbamate correspondant par réaction avec un réactif de type chloroformiate d'alkyle, comme par exemple le chloroformiate d'éthyle, dans la pyridine. Le composé ainsi obtenu est soumis à l'action du réactif acétylénique en présence d'un complexe de PdII, d'iodure cuivreux et d'une base, comme la triéthylamine par exemple, à 80-100°C pendant quelques heures. Le composé arylacétylénique correspondant ainsi obtenu est soumis à l'action d'un alcoolate de sodium dans le solvant alcoolique correspondant, comme par exemple l'éthanolate de sodium dans l'éthanol, au reflux. Après extraction et purification par chromatographie liquide sur colonne, le dérivé indolique est obtenu.

L'alkylation et la (ou les) substitution(s) électrophile(s) sont réalisées de la même façon que pour la procédure 1.

Les exemples 12 à 16 illustrent cette procédure générale 3.

Les composés de formule générale I dans laquelle : A, B, C, ainsi que $R^1$ à $R^6$ sont définis comme précédemment, étant entendu que

- lorsque A ou C représente H et l'autre substituant C ou A représente

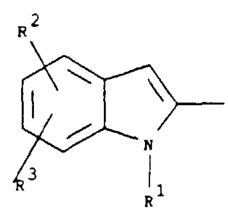

$R^2$ et $R^3$ sont différents de H; $-OR^4$ ; et

- Lorsque A ou C représente H, l'autre substituant C ou A représente

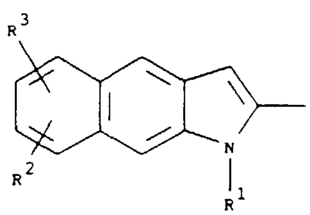

et B représente

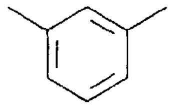

$R^1$, $R^2$ et $R^3$ ne représentent pas simultanément H,
sont nouveaux et constituent l'un des objets de la présente invention.

L'invention sera expliquée plus en détail à l'aide des exemples non limitatifs donnés dans la partie expérimentale qui suit.

**PARTIE EXPERIMENTALE:**

I. Préparation des composés utilisés selon l'invention:

Toutes les réactions sont effectuées sous atmosphère inerte d'azote, sauf indication contraire.
Les spectres de masse (SM) sont enregistrés sur un appareil Nermag R10-10B. Le mode d'ionisation utilisé est soit l'impact électronique (IE) à 70 électrons-volts, soit l'ionisation chimique (IC) dans l'ammoniac, soit le bombardement par atomes rapides (FAB) sur une matrice glycérol ou triéthanolamine.
Les spectres RMN $^1H$ et $^{13}C$ sont enregistrés sur un appareil Varian Gemini-200. Les déplacements chimiques sont exprimés en ppm par rapport au tétraméthylsilane. Les multiplicités sont exprimées comme suit : "s" pour singulet, "s1" pour singulet large, "d" pour doublet, "t" pour triplet, et "m" pour multiplet.
Les points de fusion (pF°C) sont enregistrés sur un appareil Gallenkamp et sont donnés non corrigés.
Les purifications par chromatographie liquide sur colonne sont effectuées soit avec de la silice Merck® Si60, soit avec de l'alumine neutre d'activité II-III Merck®.

A. Exemples utilisant la procédure 1:

**Exemple 1** : Préparation du 1,1'-diméthyl-2,2'-(m-phénylène)-diindole:

Bis-phénylhydrazone du 1,3-diacétylbenzène:

Du 1,3-diacétylbenzène (6,48 g; 40 mmoles) en solution dans 20 ml d'éthanol est ajouté à une solution de chlorhydrate de phénylhydrazine (12,72 g; 88 mmoles) et d'acétate de sodium (19,68 g; 240 mmoles) dans 80 ml d'eau, au reflux. Le mélange réactionnel est maintenu dans ces conditions 30 minutes après la fin de l'addition. Après refroidissement à température ambiante, il est agité 3 h à 0°C. La suspension ainsi obtenue est filtrée. Le précipité est lavé avec 2x50 ml d'eau, puis séché sous vide. Le produit désiré est obtenu après recristallisation (éthanol à 95 %).
Rendement:13,41 g; 98 %.
Caractéristiques physiques :

- RMN $^1H$(DMSO-$d_6$):
  2,27 ppm (s, 6H); 6,78 ppm (m, 2H): 7,20-7,32 ppm (m. 8H); 7,40 ppm (t, 1H, J=8 Hz); 7,68 ppm (d, 2H, J=8 Hz); 8,29 ppm (sl, 1H); 9,31 ppm (s, 2H).

2,2'-(m-phénylène)-diindole:

Le produit obtenu ci-dessus est dissous à 90°C dans 100 ml d'acide polyphosphorique. Le mélange ainsi obtenu est chauffé pendant 3 h à 120-130°C. Puis le mélange réactionnel est versé dans 800 ml d'eau-glace; la dissolution complète de l'acide nécessite environ 1,5 h. Un précipité apparaît au cours de cette étape. La suspension obtenue est filtrée puis lavée avec 2x200 ml d'eau. Le résidu est redissous dans 400 ml d'acétate d'éthyle et lavé avec une solution saturée de chlorure de sodium jusqu'à neutralité. La phase organique est séchée sur sulfate de magnésium. Le solvant est évaporé sous pression réduite. Le produit désiré est obtenu après purification par chromatographie liquide sur colonne d'alumine (gradient d'élution : hexane-acétate d'éthyle 6:1 à 4:1).
Rendement : 3,85 g; 32 %.
Caractéristiques physiques :

* Point de fusion :282°C déc.
* RMN $^1H$(DMSO-$d_6$):
6,98-7,18 ppm (m, 6H), 7,44 ppm (d, 2H, J=8 Hz); 7,55 ppm (t, 1H, J=7 Hz); 7,58 ppm (d, 2H, J=8 Hz); 7,80 ppm (dd, 2H, J=1,5-7 Hz); 8,39 ppm (t, 1H, J=1,5 Hz).

1,1'-diméthyl-2,2'-(m-phénylène)-diindole :

A une solution du 2,2'-(m-phénylène)-diindole obtenu ci-dessus (3,85 g; 12,5 mmoles) dans 30 ml de diméthylsulfoxyde, est ajoutée de la potasse en poudre (5,6 g; 100 mmoles). Le mélange réactionnel est agité pendant 64 h à température ambiante. Puis du sulfate de méthyle (15,75 g; 125 mmoles) est ajouté. Après 15 minutes, le mélange réactionnel est additionné de 100 ml d'eau. Le produit est extrait par 300 ml d'acétate d'éthyle. La phase organique est lavée par 2x100 ml d'une solution saturée de chlorure de sodium, puis séchée sur sulfate de magnésium. Le produit désiré est obtenu après recristallisation (hexane-acétate d'éthyle 1:1).
Rendement : 3,33 g; 81 %.
Caractéristiques physiques :

* Point de fusion : 150°C.
* RMN $^1H$ (DMSO-$d_6$) :
3,83 ppm (s, 6H); 6,68 ppm (s, 2H); 7,08 ppm (m, 2H); 7,21 ppm (m, 2H); 7,52 ppm (d, 2H, J=8 Hz); 7,59 ppm (d, 2H, J=7,5 Hz); 7,67 ppm (s, 3H); 7,79 ppm (s, 1H).
* RMN $^{13}C$ (DMSO-$d_6$) :
31,25 ppm; 101,84 ppm; 110,47 ppm; 119,95 ppm; 120,45 ppm; 121,90 ppm; 127,69 ppm; 127,71 ppm; 128,79 ppm; 129,51 ppm; 132,95 ppm; 138,56 ppm; 140,83 ppm.
* SM (IE, 70eV) :
336 (100, M+); 320 (12): 204 (10); 168 (30).

**Exemple 2**: Préparation du 5-fluoro-1-méthyl-2-(2'-thiényl)-indole:

4-fluorophénylhydrazone du 2-acétylthiophène :

Ce composé est préparé à partir du chlorhydrate de la 4-fluorophénylhydrazine commerciale (Aldrich) et du 2-acétylthiophène commercial (Aldrich), selon la procédure 1.
Rendement : 71 %.
Caractéristiques physiques :

* RMN $^1H$ (DMSO-$d_6$) :
2,25 ppm (s,3H); 6,98-7,20 ppm (m, 5H); 7,23 ppm (d, 1H, J=3,0 Hz); 7,40 ppm (d, 1H, J=5,0 Hz); 9,30 ppm (sl, 1H).

5-fluoro-2-(2'-thiényl)-indole:

Ce composé est préparé à partir de la 4-fluoro-phénylhydrazone du 2-acétylthiophène précédemment obtenue, selon la procédure 1.
Rendement : 46 %.
Caractéristiques physiques :

* RMN $^1H$ (DMSO-$d_6$) :
6,65 ppm (s, 1H); 6,93 ppm (td, 1H, J=2,5-9,0-9,0 Hz); 7,15 ppm (dd, 1H, J=3,5-5,0 Hz); 7,25 ppm (dd, 1H, J=2,5-10,0 Hz); 7,34 ppm (dd, 1H, J=4,5-9,0 Hz); 7,50-7,58 ppm (m, 2H); 11,66 ppm (sl, 1H).

5-fluoro-1-méthyl-2-(2'-thiényl)-indole:

Ce composé est préparé à partir du 5-fluoro-2-(2'-thiényl)-indole précédemment obtenu, selon la procédure 1.
Rendement : 74 %.
Caractéristiques physiques :

* RMN $^1H$ (DMSO-$d_6$) :
3,85 ppm (s, 3H); 6,65 ppm (s, 1H); 7,15 ppm (td, 1H, J=2,5-9,0-9,0 Hz); 7,23 ppm (dd, 1H, J=3,5-5,0 Hz); 7,35 ppm (dd, 1H, J=2,5-10,0 Hz); 7,43 ppm (d, 1H, J=3,5 Hz); 7,53 ppm (dd, 1H, J=4,5-9,0 Hz); 7,70 ppm (d, 1H, J=5,0

Hz).

* RMN $^{13}C$ (DMSO-$d_6$) :
31,32 ppm; 101,98 ppm (d, J=5,2 Hz); 104,82 ppm (d, J=23,5 Hz); 110,16 ppm (d, J=26,1 Hz); 111,59 ppm (d, J=10,1 Hz); 127,49 ppm; 127,54 ppm; 128,53 ppm; 133,26 ppm; 135,14 ppm: 135,64 ppm; 157,7 ppm (d, J=233,4 Hz).
* SM (IE, 70eV) :
231(100, M+); 216 (14): 107 (12); 45 (20).

**Exemple 3** : Préparation du 4- et du 6-fluoro-1-méthyl-2-(2'-thiényl)-indole:

3-fluorophénylhydrazone du 2-acétylthiophène :

Ce composé est préparé à partir du chlorhydrate de la 3-fluorophénylhydrazine commerciale (Aldrich) et du 2-acétylthiophène commercial (Aldrich), selon la procédure 1.
Rendement : 42 %.
Caractéristiques physiques :

* RMN $^{1}H$ (DMSO-$d_6$) :
2,26 ppm (s, 3H); 6,52 ppm (td, 1H, J=2,5-8,5-8,5 Hz); 6,84-7,0 ppm (m, 2H); 7,03 ppm (dd, 1H, J=3,5-5,0 Hz); 7,16-7,30 ppm (m, 1H); 7,28 ppm (dd, 1H, J=1,0-3,5 Hz); 7,43 ppm (dd, 1H, J=1,0-5,0 Hz); 9,50 ppm (sl, 1H).

4- et 6-fluoro-2-(2'-thiényl)-indole:

Ces composés sont préparés à partir de la 3-fluorophénylhydrazone du 2-acétylthiophène obtenue précédemment, selon la procédure 1.
Rendement : 74 % d'un mélange 1:1 des deux isomères.
Caractéristiques physiques :

* RMN $^{1}H$ (DMSO-$d_6$) : mélange 1:1 des deux isomères :
6,68 ppm (d, 0,5H, J=1 Hz); 6,72 ppm (m, 0,5H); 6,76-6,91 ppm (m, 1H); 7,0-7,24 ppm (m, 2,5H); 7,44-7,60 ppm (m, 2,5 H); 11,7 ppm (sl, 0,5H); 11,9 ppm (sl, 0,5H).

4- et 6-fluoro-1-méthyl-2-(2'-thiényl)-indole:

Ces composés sont préparés à partir du mélange du 4- et du 6-fluoro-2-(2'-thiényl)-indole obtenu précédemment, selon la procédure 1.
Rendement : 68 %.

4-fluoro-1-méthyl-2-(2'-thiényl)-indole:

Caractéristiques physiques :

* Point de fusion : 86°C
* RMN $^{1}H$ (DMSO-$d_6$) :
3,85 ppm (s, 3H); 6,70 ppm (s, 1H); 6,85 ppm (dd, 1H, J=8,0-10,0 Hz); 7,17 ppm (td, 1H, J=5,0-8,0-8,0 Hz); 7,24 ppm (dd, 1H, J=3,5-5,0 Hz); 7,37 ppm (d, 1H, J=8,5 Hz); 7,47 ppm (d, 1H, J=3,5 Hz); 7,52 ppm (d, 1H, J=5,0 Hz).
* RMN $^{13}C$ (DMSO-$d_6$) :
31,72 ppm; 99,06 ppm; 105,21 ppm (d, J=19,2 Hz); 106,17 ppm (d, J=3,5 Hz); 122,97 ppm (d, J=7,4 Hz); 127,03 ppm; 127,06 ppm; 127,66 ppm; 128,17 ppm; 128,21 ppm; 134,28 ppm (d, J=24,1 Hz); 141,43 ppm (d, J=12 Hz); 156,77 ppm (d, J=248 Hz).
* SM (IE, 70eV) :
231(100, M+); 216 (8); 198 (10); 185 (14); 172 (10); 45 (20).

6-fluoro-1-méthyl-2-(2'-thiényl)-indole :

Caractéristiques physiques :

* Point de fusion : 74-75°C:

* RMN $^1H$ (DMSO-$d_6$) :
3,80 ppm (s, 3H); 6,92 ppm (td, 1H, J=2,5-9,0-9,0 Hz); 7,21 ppm (dd, 1H, J=3,5-5,0 Hz); 7,34-7,44 ppm (m, 2H); 7,54 ppm (dd, 1H, J=5,5-8,5 Hz); 7,67 ppm (d, 1H; J=5,0 Hz).
* RMN$^{13}C$ (DMSO-$d_6$) :
31,44 ppm; 96,49 ppm (d, J=26,5 Hz); 103,26 ppm; 109,23 ppm (d, J=24,5 Hz); 121,84 ppm (d, J=10,0 Hz); 124,67 ppm; 126,76 ppm; 127,29 ppm; 128,20 ppm; 134,40 ppm; 134,99 ppm (d, J=4,0 Hz); 139,00 ppm (d, J=11,8 Hz); 160,66 ppm (d, J=238,9 Hz).
* SM (IE, 71eV) :
231(100, M+); 216 (10); 185 (12); 172 (14); 58 (50); 45 (60).

**Exemple 4** : Préparation du 1-méthyl-2-(2'-sélénophényl)-indole :

Phénylhydrazone du 2-acétylsélénophène:

Ce composé est préparé à partir du chlorhydrate de la phénylhydrazine commerciale (Aldrich) et du 2-acétyl-sélénophène, lui-même obtenu selon la procédure décrite par S. Umezawa (référence 18).
Rendement : 82 %.
Caractéristiques physiques :

* RMN $^1H$ (DMSO-$d_6$) :
2,25 ppm (s, 3H); 6,75 ppm (tt, 1H, J=1,5-1,5-7,0-7,0 Hz); 7,08-7,28 ppm (m, 4H); 7,24 ppm (dd, 1H, J=3,5-5,5 Hz); 7,35 ppm (dd, 1H, J=1,0-3,5 Hz); 7,95 ppm (dd, 1H, J=1,0-5,5 Hz); 9,30 ppm (sl, 1H).

2-(2'-sélénophényl)-indole:

Ce composé est préparé à partir de la phényl-hydrazone du 2-acétylsélénophène obtenue précédemment, selon la procédure 1.
Rendement : 61 %.
Caractéristiques physiques :

* RMN $^1H$ (DMSO-$d_6$) :
6,66 ppm (d, 1H, J=1,5 Hz); 6,97 ppm (m, 1H); 7,09 ppm (m, 1H); 7,33 ppm (dd, 1H, J=4,0-5,5 Hz); 7,34 ppm (d, 1H, J=8,0 Hz); 7,48 ppm (d, 1H, J=8,0 Hz); 7,65 ppm (dd, 1H, J=1,0-4,0 Hz); 8,11 ppm (dd, 1H, J=1,0-5,5 Hz); 11,51 ppm (sl, 1H).
* RMN $^{13}C$ (DMSO-$d_6$) :
99,92 ppm; 111,39 ppm; 119,82 ppm; 120,15 ppm; 122,11 ppm; 125,68 ppm; 128,87 ppm; 130,66 ppm; 130,98 ppm; 134,73 ppm; 137,21 ppm; 140,99 ppm.
* SM (IE,70eV) :
247 (100, M+); 167 (80); 139 (40); 127 (15); 113 (15); 89 (30); 63 (40); 43 (45).

1-méthyl-2-(2'-sélénophényl)-indole:

Ce composé est préparé à partir du 2-(2'-séléno-phényl)-indole obtenu précédemment, selon la procédure 1.
Rendement : 90 %.
Caractéristiques physiques :

* RMN $^1H$ (DMSO-$d_6$) :
3,83 ppm (s, 3H); 6,65 ppm (s, 1H); 7,05 ppm (m, 1H); 7,20 ppm (m, 1H); 7,43 ppm (dd, 1H, J=3,5-5,0 Hz); 7,45-7,57 ppm (m, 3H); 8,28 ppm (dd, 1H, J=1,0-5,0 Hz).
* RMN $^{13}C$ (DMSO-$d_6$) :
31,04 ppm; 102,53 ppm; 110,41 ppm; 120,09 ppm; 120,33 ppm; 122,15 ppm; 127,50 ppm; 129,24 ppm; 130,76 ppm; 133,29 ppm; 135,89 ppm; 138,47 ppm; 139,06 ppm.
* SM (IE, 70eV) :
261 (100, M+); 180 (70); 167 (20); 152 (25); 63 (30).

**Exemple 5** : Préparation du 1-méthyl-2-phénylindole :

Ce composé est obtenu à partir du 2-phénylindole commercial (Lancaster) par une réaction d'alkylation selon

la procédure 1.
Rendement : 87 %.
Caractéristiques physiques :

* Point de fusion : 100-101°C.
* RMN $^1H$(DMSO-$d_6$):
 3,75 ppm (s, 3H); 6,57 ppm (s, 1H); 7,07 ppm (m, 1H); 7,19 ppm (m, 1H); 7,40-7,64 ppm (m, 6H).
* RMN $^{13}C$ (DMSO-$d_6$) :
 31,05 ppm; 101,43 ppm; 110,37 ppm; 119,90 ppm; 120,40 ppm; 121,75 ppm; 127,78 ppm; 128,21 ppm; 128,97 ppm; 129,30 ppm; 132,54 ppm; 138,49 ppm; 141,53 ppm.
* SM (IE, 70eV) :
 207 (100, M+).

**Exemple 6 :** Préparation du 5-(1-méthyl-2-phényl-indolyl)-sulfonate de sodium :

Ce composé est préparé à partir du 1-méthyl-2-phénylindole obtenu précédemment, selon la procédure classique (référence 15).
Rendement : 24 %.
Caractéristiques physiques :

* Point de fusion : >360°C.
* RMN $^1H$ ($D_2O$) :
 3,18 ppm (s, 3H); 6,21 ppm (s, 1H); 7,0-7,20 ppm (m, 6H); 7,55 ppm (dd, 1H, J=1,5-8,0 Hz); 7,90 ppm (d, 1H, J=1,5 Hz).
* RMN $^{13}C$ ($D_2O$) :
 32,94 ppm; 104,62 ppm; 113,00 ppm; 120,89 ppm; 121,69 ppm; 129,07 ppm; 130,51 ppm; 130,98 ppm; 131,38 ppm; 134,23 ppm; 137,22 ppm; 141,66 ppm; 145,09 ppm.

**Exemple 7 :** Préparation du 5,5'-[1,1'-diméthyl-2,2'-(m-phénylène)-diindolyl]disulfonate de sodium :

Ce composé est préparé à partir du 1,1'-diméthyl-2,2'-(m-phénylène)-diindole obtenu à l'exemple 1, selon la procédure classique (référence 15). Rendement : 48 %.
Caractéristiques physiques :

* Point de fusion : >360°C.
* RMN $^1H$ ($D_2O$) :
 3,26 ppm (s, 6H); 6,13 ppm (s, 2H); 6,77 ppm (sl, 1H); 7,16-7,34 ppm (m, 5H); 7,55 ppm (dd, 2H, J=1,5-8,5 Hz); 7,95 ppm (d, 2H, J=1,5 Hz).
* RMN $^{13}C$ ($D_2O$) :
 33,11 ppm; 104,85 ppm; 113,17 ppm; 120,77 ppm; 121,57 ppm; 128,89 ppm; 128,98 ppm; 131,06 ppm; 131,59 ppm; 134,33 ppm; 136,87 ppm; 141,71 ppm; 144,71 ppm; 144,76 ppm; 144,85 ppm.
* SM (FAB, glycérol) ;
 563 (20); 541 (M+H+, 20): 461 (20); 265 (100).

B. Exemples utilisant la procédure 2 :

**Exemple 8** : Préparation du 1-méthyl-2-(2'-thiényl)-indole:

2-[2'-(2"-thiényl)-éthynyl]-aniline :

A une solution de 2-éthynylaniline (440 mg; 3,8 mmoles) et de 2-iodothiophène (798 mg; 3,8 mmoles) dans 10 ml de diéthylamine-diméthylformamide (4:1) sont ajoutés du palladium tétrakis-triphénylphosphine (420 mg; 38 µmoles) et de l'iodure cuivreux (14,4 mg; 76 µmoles). Le mélange réactionnel est agité pendant 6 h à température ambiante, puis additionné de 100 ml d'eau et additionné de 100 ml de terbutylméthyl éther. La phase organique est séchée sur sulfate de sodium et filtrée. Le solvant est évaporé sous pression réduite. Le produit désiré est obtenu, sous forme cristalline, après purification par chromatographie liquide sur colonne de silice (éluant : hexane-acétate d'éthyle 9:1).
Rendement : 560 mg; 74 %.

Caractéristiques physiques :

* RMN $^1H(CDCl_3)$ :
4,23 ppm (sl, 2H); 6,70 ppm (m, 2H); 7,00 ppm (dd; 1H, J=3,5-5,0 Hz); 7,13 ppm (m, 1H); 7,22-7,38 ppm (m, 3H).

2-(2'-thiényl)-indole:

Le produit précédemment obtenu (560 mg; 2,8 mmoles) est dissous dans 15 ml d'acétonitrile puis du chlorure de palladium (25 mg; 0,14 mmole) est ajouté. Le mélange réactionnel est chauffé pendant 3 h au reflux. Le solvant est évaporé sous pression réduite. Le produit désiré est obtenu, sous forme d'une poudre légèrement jaunâtre, après purification par chromatographie liquide sur colonne de silice (éluant : hexane-acétate d'éthyle 9:1).
Rendement : 400 mg; 71 %.
Caractéristiques physiques :

* Point de fusion : 168°C.
* RMN $^1H$ (DMSO-$d_6$) :
6,65 ppm (s, 1H); 6,92 ppm (td, 1H, J=1,0-7,0-7,0 Hz); 7,09 ppm (td, 1H, J=1,0-7,0-7,0 Hz); 7,36 ppm (d, 1H, J=7,0 Hz); 7,46-7,55 ppm (m, 3H); 11,55 ppm (sl, 1H).
* RMN $^{13}C$ (DMSO-$d_6$) :
98,96 ppm; 111,40 ppm; 119,84 ppm; 120,20 ppm; 122,04 ppm; 123,79 ppm; 125,41 ppm; 128,43 ppm; 128,80 ppm; 132,22 ppm; 135,85 ppm; 137,13 ppm.
* SM (IE, 70eV) :
199 (100, M+); 171(10); 154 (10).

1-méthyl-2-(2'-thiényl)-indole:

A une solution de 2-(2'-thiényl)-indole (3,0 g; 15 mmoles) dans 30 ml de diméthylsulfoxyde est ajoutée de la potasse en poudre (2,52 g; 45 mmoles). Le mélange réactionnel est agité pendant 14 h à température ambiante. Puis du sulfate de méthyle (6,6 g; 52,5 mmoles) est ajouté. Après 15 minutes, le mélange réactionnel est additionné de 100 ml d'eau. Le produit est extrait par 200 ml d'acétate d'éthyle. La phase organique est lavée par 2x100 ml d'une solution saturée de chlorure de sodium, puis séchée sur sulfate de magnésium. Le produit désiré est obtenu après purification par chromatographie liquide sur colonne de silice (éluant : cyclohexane-acétate d'éthyle 30:1) et recristallisation (hexane-isopropanol 2:1).
Rendement : 2,62 g; 82 %.
Caractéristiques physiques :

* Point de fusion :58°C.
* RMN $^1H$ (DMSO-$d_6$) :
3,87 ppm (s, 3H): 6,65 ppm (s, 1H); 7,05 ppm (t, 1H, J=8,0 Hz); 7,19 ppm (t, 1H, J=8,0 Hz); 7,23 ppm (dd, 1H J=3,5-5,0 Hz); 7,41 ppm (d, 1H, J=3,5 Hz); 7,50 ppm (d, 1H, J=7,0 Hz); 7.55 ppm (d. 1H. J=7,(1 Hz); 7,68 ppm (d, 1H, J=5,0 Hz).
* RMN $^{13}C$ (DMSO-$d_6$) :
31,03 ppm; 102,04 ppm; 110,39 ppm; 120,08 ppm; 120,37 ppm; 122,14 ppm; 127,17 ppm; 127,43 ppm; 128,53 ppm; 133,65 ppm; 133,87 ppm; 138,37 ppm.
* SM (IE, 70eV):
213 (100, M+); 167 (15); 154 (15); 89 (20); 58 (60); 45 (45); 39 (25).

**Exemple 9** : Préparation du 1-méthyl-5-nitro-2-(2'-thiényl)-indole :

5-nitro-2-(2'-thiényl)-indole:

Ce composé est obtenu par nitration du 2-(2'-thiényl)-indole selon la procédure décrite dans la référence 19.
Rendement : 53 %.
Caractéristiques physiques :

* RMN $^1H$ (DMSO-$d_6$) :
6,96 ppm (s, 1H); 7,20 ppm (dd, 1H, J=3,5-5,0 Hz); 7,52 ppm (d, 1H, J=9,0 Hz); 7,60-7,66 ppm (m, 2H); 8,0 ppm (dd, 1H, J=2,0-9,0 Hz); 8,51 ppm (d, J=2 Hz); 12,33 ppm (sl, 1H).

1-méthyl-5-nitro-2-(2'-thiényl)-indole:

Ce composé est obtenu à partir du 5-nitro-2-(2'-thiényl)-indole obtenu précédemment, par une réaction d'alkylation selon la procédure 1.
Rendement: 83 %.

* RMN $^1H$ (DMSO-$d_6$) :
  3,98 ppm (s, 3H); 6,98 ppm (s, 1H); 7,27 ppm (dd, 1H, J=3,5-5,5 Hz); 7,53 ppm (d, 1H, J=3,5 Hz); 7,74 ppm (d, 1H, J=9,0 Hz); 7,78 ppm (d, 1H, J=5,5 Hz); 8,07 ppm (dd, 1H, J=2,0-9,0 Hz); 8,57 ppm (d, 1H, J=2,0 Hz).

**Exemple 10** : Préparation du 5-amino-1-méthyl-2-(2'-thiényl)-indole:

Ce composé est obtenu à partir du 1-méthyl-5-nitro-2-(2'-thiényl)-indole obtenu à l'exemple 9, par réduction au moyen de chlorure d'étain ($SnCl_2$). Rendement : 63 %.
Caractéristiques physiques :

* RMN $^1H$ (DMSO-$d_6$) :
  3,72 ppm (d, 3H, J=1,5 Hz); 4,60 ppm (sl, 2H), 6,35 ppm (s, 1H): 6,58 ppm (d, 1H, J=8,5 Hz); 6,65 ppm (s, 1H); 7,14-7,22 ppm (m, 3H); 7,32 ppm (m, 1H); 7.61 ppm (m, 1H).
* SM (IE, 70eV) :
  228 (100, M+); 213 (30); 114 (10).

**Exemple 11** : Préparation du 2-{1'-[2'-(2"-thiényl)-indolyl]}-éthylphosphonate disodique :

1-(2'-diéthoxyphosphono)éthyl-2-(2"-thiényl)-indole :

Ce composé est obtenu par alkylation du 2-(2'-thiényl)-indole au moyen de diéthoxyphosphonate de vinyle commercial (Aldrich), selon la procédure d'alkylation utilisée dans les exemples précédents.
Rendement : 53 %.
Caractéristiques physiques :

* RMN $^1H$ (DMSO-$d_6$) :
  1,17 ppm (t, 6H, J=7,0 Hz); 2,17 ppm (m, 2H); 3,94 ppm (m, 4H); 4,46 ppm (m, 2H); 6,66 ppm (s, 1H); 7,08 ppm (t, 1H, J=7,5 Hz); 7,17-7,27 ppm (m, 1H); 7,47 ppm (d, 1H, J=7,5 Hz); 7,57 ppm (d, 1H, J=7,5 Hz); 7,72 ppm (d, 1H, J=5,0 Hz).

2{[1'-[2'-(2"-thiényl)-indolyl]}éthylphosphonate disodique:

Ce composé est obtenu par hydrolyse du 1-(2'-diéthoxyphosphono)éthyl-2-(2"-thiényl)-indole précédent, au moyen de bromure de triméthylsilyle. Rendement : 99 %.
Caractéristiques physiques :

* RMN $^1H$ ($D_2O$) :
  1,85 ppm (m, 2H); 4,42 ppm (m, 2H); 6,48 ppm (s, 1H); 7,02 ppm (t, 1H, J=7,5 Hz); 7,09 ppm (dd, 1H, J=3,5-5,0 Hz); 7,16-7,26 ppm (m, 2H); 7,39 ppm (dd, 1H, J=1,0-5,0 Hz); 7,45 ppm (d, 1H; J=7,5 Hz); 7,53 ppm (d, 1H, J=7,5 Hz).
* RMN $^{13}C$ ($D_2O$) :
  36,75 ppm (d, J=125 Hz); 42,94 ppm; 105,01 ppm; 113,35 ppm; 123,10 ppm; 123,48 ppm; 125,16 ppm; 129,79 ppm: 130,38 ppm; 131,10 ppm; 136,10 ppm; 136,55 ppm; 139,99 ppm.
* SM (FAB, glycérol) : 374 (55); 352 (30, M+H+); 165 (25); 148 (30); 137 (50); 122 (70); 108 (100).

C. Exemple utilisant la procédure 3:

**Exemple 12** : Préparation du 1-méthyl-2-phénylbenz(f)indole :

3-amino-2-bromo-naphtalène :

Ce composé est obtenu à partir du bis(hexa-chlorocyclopentadiényl)-2-bromo-3-nitronaphtalène commercial

(Aldrich), selon la procédure décrite dans la référence 20.
Rendement : 71 %.
Caractéristiques physiques :

* RMN $^1H(CDCl_3)$ :
4,25 ppm (sl, 2H); 7,09 ppm (s, 1H); 7,22 ppm (t, 1H, J=7,5 Hz); 7,36 ppm (t, 1H, J=7,5 Hz); 7,56 ppm (d, 1H, J=7,5 Hz); 7,60 ppm (d, 1H, J=7,5 Hz); 7,96 ppm (s, 1H).

<u>2-bromo-3-(éthoxycarbonyl)amino-naphtalène :</u>

Ce composé est préparé à partir du 3-amino-2-bromo-naphtalène, obtenu précédemment, et du chloroformiate d'éthyle commercial (Aldrich), selon la procédure 3.
Rendement : 75 %.
Caractéristiques physiques :

* RMN $^1H(CDCl_3)$ :
1,36 ppm (t, 3H, J=7 Hz); 4,28 ppm (q, 2H, J=7 Hz); 7,29 ppm (sl, 1H); 7,42 ppm (m, 2H); 7,66 ppm (d, 1H, J=8 Hz); 7,78 ppm (d, 1H, J=8 Hz); 8,05 ppm (s, 1H); 8,59 ppm (s, 1H).
* SM (IE, 70eV) :
293 (32, M+); 249 (12); 234 (25); 223 (35); 214 (14); 193 (70); 186 (77); 140 (35); 114 (100); 87 (28); 63 (50).

<u>2-(éthoxycarbonyl)amino-3-(2'-phényléthynyl)-naphtalène :</u>

Ce composé est préparé à partir du 2-bromo-3-(éthoxycarbonyl)aminonaphtalène obtenu ci-dessus, selon la procédure 3.
Rendement : 83 %.
Caractéristiques physiques :

* RMN $^1H(CDCl_3)$ :
1,37 ppm (t, 3H, J=7 Hz); 4,30 ppm (q, 2H, J=7 Hz); 7,32-7,50 ppm (m, 5H); 7,54-7,63 ppm (m, 3H); 7,72 ppm (d, 1H, J=8 Hz); 7,78 ppm (d, 1H, J=8 Hz); 8,02 ppm (s, 1H); 8,58 ppm (s, 1H).

<u>2-phényl-1(H)-benz(f)indole :</u>

Ce composé est préparé à partir du 2-(éthoxy-carbonyl)amino-3-(2'-phényléthynyl)-naphtalène obtenu ci-dessus, selon la procédure 3.
Rendement : 34 %.
Caractéristiques physiques :

* RMN $^1H$ (DMSO-$d_6$) :
7,08 ppm (d, 1H, J=1 Hz); 7,27 ppm (m, 2H); 7,40 ppm (d, 1H, J=7 Hz); 7,51 ppm (t, 2H, J=7,5 Hz); 7,85 ppm (s, 1H); 7,87-8,09 ppm (m, 4H); 8,07 ppm (s, 1H); 11,54 ppm (sl, 1H).
* SM (IE, 70eV) :
243 (100, M+); 215 (18); 139 (38); 87 (13); 77 (25); 63 (14).

<u>1-méthyl-2-phénylbenz(f)indole :</u>

Ce composé est préparé à partir du 2-phényl-1(H)-benz(f)indole par alkylation, comme décrit dans la procédure 1.
Rendement : 72 %.
Caractéristiques physiques :

* RMN $^1H$ (DMSO-$d_6$) :
3,85 ppm (s, 3H); 6,75 ppm (s, 1H); 7,33 ppm (m, 2H); 7,47-7,62 ppm (m, 3H); 7,65-7,72 ppm (m, 2H); 7,91-8,02 ppm (m, 3H); 8,13 ppm (s, 1H).
* SM (IE,70 eV) :
257 (100, M+); 215 (12); 77 (11).

**Exemple 13**: Préparation de l'acide 1-méthyl-2-phényl-benz(f)indole-5,7-disulfonique:

Le dérivé 1-méthyl-2-phénylbenz(f)indole, obtenu précédemment, (0,15g; 0,58mmoles) est traité avec 1ml d'oléum (20%$SO_3$) pendant 1h à 0°C. Puis le mélange réactionnel est additionné de 2ml d'eau froide, ajoutés lentement goutte à goutte. Après filtration de la suspension, ainsi obtenue, et lavage avec 200 µl d'eau froide, le mélange des deux isomères, l'acide 1-méthyl-2-phényl-benz(f)indole-5,7-disulfoniquc et l'acide 1-méthyl-2-phényl-benz(f)indole-6,8-disulfonique, est obtenu sous forme d'une poudre jaune très hygroscopique.Rendement du mélange: 88%.

Le produit désiré est obtenu, après purification par chromatographie liquide sur colonne, avec un rendement de 35%.
Caractéristiques physiques:

* RMN $^1H$ ( $D_2O$+NaOD):
  3,44 ppm (s; 3H); 7,30 ppm (m; 5H); 7,84 ppm (s; 1H); 8,21 ppm (m; 1H); 8,45 ppm (m; 1H); 8,80 ppm (s; 1H).
* SM (FAB négatif; triéthanolamine):
  416 (M-H; 50%); 336 (20%); 195 (100%).

**Exemple 14**: Préparation de l'acide 1-méthyl-2-phényl-benz(f)indole-6,8-disulfonique:

Ce dérivé est obtenu à partir du mélange précédent, après purification par chromatographie liquide sur colonne, avec un rendement de 53%.
Caractéristiques physiques:

* RMN $^1H$ ( $D_2O$+NaOD):
  3,61 ppm (s; 3H); 7,30 ppm (m; 5H); 8,08 ppm (s; 1H); 8,26 ppm (m; 1H); 8,43 ppm (m; 1H); 8,50 ppm (s; 1H).
* SM (FAB négatif; triéthanolamine):
  416 (M-H; 50%); 336 (20%); 195 (100%).

**Exemple 15** : Préparation du sel disodique de l'acide 1-méthyl-2-phénylbenz(f)indole-5,7-disulfonique:

L'acide 1-méthyl-2-phényl-benz(f)indole-5,7-disulfonique (75mg; 0,18mmoles) est traité avec 7,5ml d'une solution saturée de chlorure de sodium à chaud. Le produit désiré est obtenu après refroidissement à température ambiante, filtration et lavage avec 500µl d'une solution saturée de chlorure de sodium diluée au 4/5e sous forme de cristaux plats légèrement jaunes et fluorescents. Rendement: 78%
Caractéristiques physiques:

* pF: >360°C
* RMN $^1H$ ($D_2O$): 3,60 ppm (s; 3H); 6,65 ppm (sl; 1H); 7,38-7,50 ppm (m; 5H); 7,98 ppm (s; 1H); 8,19 ppm (m; 1H); 8,50 ppm (m; 1H); 8,82 ppm (s; 1H).
* SM (FAB négatif; triéthanolamine):
  496 (20%); 438 (35%); 206 (100%); 194 (85%); 171(46%); 113(45%).

**Exemple 16:** Préparation du sel disodique de l'acide 1-méthyl-2-phénylbenz(f)indole-6,8-disulfonique:

L'acide 1-méthyl-2-phényl-benz(f)indole-6,8-disulfonique (80mg; 0,19mmoles) est traité avec 8,0ml d'une solution saturée de chlorure de sodium à chaud. Le produit désiré est obtenu après refroidissement à température ambiante, filtration et lavage avec 500µl d'une solution saturée de chlorure de sodium diluée au 4/5e sous forme de cristaux plats légèrement jaunes et fluorescents. Rendement: 82%
Caractéristiques physiques:

* pF: >360°C
* RMN $^1H$ ($D_2O$):
  3,73 ppm (s; 3H); 6,61 ppm (sl; 1H); 7,38-7,50 ppm (m; 5H); 8,23 ppm (sl; 2H); 8,49 ppm (sl; 1H); 8,54 ppm (s; 1H).
* SM (FAB négatif; triéthanolamine):
  496 (20%); 438 (35%); 206 (100%); 194 (85%); 171(46%); 113(45%).

**II. Exemple de mise en oeuvre du procédé selon l'invention :**

**Exemple 17** : Dosage colorimétrique de la peroxydation lipidique en utilisant le 1-méthyl-2-phénylindole (composé de l'exemple 5):

Le 1-méthyl-2-phénylindole (appelé ci-après "réactif chromogène R1" ou plus simplement "réactif R1") réagit avec le MDA et les 4-hydroxy-2-énaldéhydes à 45°C. La condensation d'une molécule de MDA ou de 4-hydroxy-2-énaldéhydes avec deux molécules de réactif R1 produit un chromophore stable dont la longueur d'onde d'absorbance maximale, caractéristique du réactif R1, est égale à 586 nm. Pour cette raison, cette méthode sera appelée ci-après "méthode LPO-586".

Dans le cas du MDA, ce chromophore a pour formule:

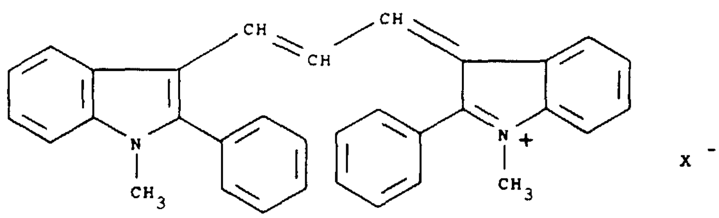

$X^- = Cl^-$ ; $CH_3SO_3^-$

La méthode LPO-586 permet un dosage spécifique du MDA et des 4-hydroxy-2-énaldéhydes sur une prise d'essai d'échantillon aqueux. Sa simplicité de mise en oeuvre est particulièrement bien adaptée à la mesure de la peroxydation lipidique sur de grandes séries d'échantillons.

La plupart des interférences connues pour d'autres méthodes de dosage colorimétrique sont éliminées dans la méthode LPO-586, ce qui se traduit par une spécificité et une sensibilité très supérieures.

Une variante de la méthode LPO-586 permet de mesurer spécifiquement la quantité de MDA dans un échantillon contenant des 4-hydroxy-2-énaldéhydes (voir plus loin).

Le mesure spécifique de ces deux paramètres dans un même échantillon permet d'évaluer l'éventuelle origine enzymatique du MDA (biosynthèse du thromboxane, par exemple).

Les principaux réactifs utilisés pour la mise en oeuvre de cette méthode peuvent être rassemblés dans un coffret (sous forme de nécessaire ou kit).

Un tel coffret peut par exemple contenir les quantités de réactifs permettant d'effectuer 100 dosages, à savoir :
Réactif R1 : solution du réactif chromogène $R^1$ 10,3 mmolaire dans l'acétonitrile; (3 x 18 ml).
Réactif R2 : acide méthanesulfonique 10,4 molaire; (3 x 5,5 ml) contenant le cas échéant du chlorure ferrique 34 μmolaire.
Solution standard S1 : solution de 1,1,3,3-tétraméthoxypropane 10 mmolaire dans du tampon Tris-HCl 20 mmolaire, pH 7,4; (1 ml).
Solution standard S2 : solution de diéthylacétal du 4-hydroxynonénal 10 mmolaire dans l'acétonitrile (1 ml).
Note : le 1,1,3,3-tétraméthoxypropane et le diéthylacétal du 4-hydroxynonénal se transforment, en milieu acide, respectivement en MDA et 4-hydroxynonénal.

Toutes ces solutions doivent être conditionnées en flacons hermétiquement fermés et doivent être conservées entre +4 et +10°C jusqu'à la date de péremption indiquée sur l'étiquette du coffret. Elles ne doivent pas être congelées.

Après ouverture et utilisation, les flacons soigneusement bouchés doivent être conservés entre +4 et +10°C.

Dans ces conditions, les solutions peuvent être utilisées pendant les deux mois qui suivent la date de première ouverture des flacons.

Le matériel utilisé est essentiellement constitué par :

- un spectrophotomètre d'absorption permettant de mesurer l'absorption à une longueur d'onde de 586 nm (+ 4 nm), sur un intervalle de 0 à 2 unités d'absorbance;
- des cuves spectrophotométriques de 1 ml et de 1 cm de trajet optique;
- un bain-marie thermostaté à 45 ± 1°C.

La mesure de peroxydation lipidique effectuée impose que la verrerie utilisée ait subi un lavage à l'acide (acétique ou chlorhydrique) et un rinçage exhaustif à l'eau désionisée très pure. Les tubes à essai et bouchons à usage

unique doivent être compatibles avec les solvants et acides du coffret.

Tous les produits (eau, tampons, solvants) utilisés pour la dilution des échantillons ou pour les mesures doivent être de qualité ultrapure.

**PROCEDURE EXPERIMENTALE**

1. Préparation de la solution finale de réactif R1.

Immédiatement avant utilisation, on dilue trois volumes de la solution initiale de réactif $R^1$ (18 ml) par addition d'un volume (6 ml) de méthanol pur.

On obtient une solution R1-dil contenant R1 7,7 mmolaire dans un mélange acétonitrile/méthanol 3:1 (v/v).

Cette solution prête à l'emploi, ne peut être conservée plus d'une journée.

2. Dosage.

Avant chaque série de mesures, on ajuste le zéro d'absorbance du spectrophotomètre à 586 nm contre de l'eau.

a) On distribue 650 µl de solution R1-dil dans tous les tubes à essai.
b) Pour chaque dosage :

On ajoute 200 µl d'échantillon et on mélange.

On démarre la réaction par l'addition de 150 µl de réactif R2 (acide méthanesulfonique). On mélange bien puis on bouche soigneusement.

On incube le mélange réactionnel pendant 40 min dans un bain-marie équilibré à 45°C.

On mesure l'absorbance (A) à 586 nm.

c) Pour chaque série de dosages, on utilise, en duplicat, un échantillon témoin ([aldéhyde] = 0) dont l'absorbance moyenne ($A_0$) sera ultérieurement soustraite des valeurs d'absorbance (A) mesurées en présence de chaque échantillon à doser.

Remarques :

Il ne faut pas inverser l'ordre d'addition des solutions.

La température de travail choisie, 45°C, permet à la réaction d'atteindre un plateau en 40 min.

L'intensité de l'absorbance à 586 nm ($A-A_0$) est une fonction linéaire de la concentration en MDA et en 4-hydroxy-2-énaldéhydes. Elle est stable au moins deux heures.

3. Etalonnage:

Les solutions standards S1 et S2 permettent respectivement de réaliser des "gammes" d'étalonnage de MDA et de 4-hydroxynonénal.

Avant emploi, on dilue ces solutions au 1/100 (v/v) dans le même milieu que l'échantillon de façon à obtenir des concentrations 100 µmolaires.

On suit la procédure 2 ci-dessus en remplaçant l'échantillon par une prise d'essai, d'un volume variant de 0 à 200 µl de solution standard (100 µmolaire), complétée à 200 µl (qsp) de milieu.

Cette procédure couvre l'intervalle des concentrations 0 à 20 µmolaires dans le milieu réactionnel final (cuve du spectrophotomètre).

Une régression linéaire des moindres carrés doit démontrer que la différence d'absorbances ($A-A_0$) est une fonction linéaire de la concentration en MDA et en 4-hydroxynonénal. S'il n'en était pas ainsi, cela signifierait que les réactifs utilisés sont impurs ou dégradés.

Le coefficient d'extinction molaire apparent (E) du produit mesuré est égal à la pente de cette droite de régression.

La figure 1 annexée montre la cinétique de formation de l'adduit du tétraméthoxypropane (TMP) avec le 1-méthyl-2-phénylindole (MPI), lors de l'incubation à 45°C avec de l'acide méthanesulfonique (réactif R2) à 15 %.

Les figures 2 et 3 fournissent des exemples de droites d'étalonnage. Elles montrent qu'on obtient le même coefficient d'extinction molaire (E) avec le MDA et le 4-hydroxynonénal.

Plus précisément :

- la figure 2 représente la courbe d'étalonnage réalisée avec le tétraméthoxypropane qui se transforme en MDA en milieu acide, établie dans les conditions suivantes : acide méthanesulfonique à 15 % (v/v), incubation à 45°C pendant 40 minutes;
- la figure 3 représente la courbe d'étalonnage réalisée avec le diéthylacétal du 4-hydroxynonénal qui se transforme en 4-hydroxynonénal en milieu acide, établie dans les mêmes conditions que la figure 2.

4. Calcul de la concentration.

L'équation suivante donne la concentration molaire en MDA et 4-hydroxy-2-énaldéhydes, tels que définis précédemment, dans l'échantillon :

$$[\text{MDA + 4-hydroxy-2-énaldéhydes}] = (A-A_0) \times 5 / E$$

Remarque : Cette concentration peut également être obtenue en reportant $A-A_0$ en ordonnée de la courbe d'étalonnage utilisée.

5. Sensibilité, précision et reproductibilité.

Sensibilité :

Le seuil de détection est inférieur à une concentration 0,5 µmolaire d'énaldéhyde dans le milieu réactionnel final.

En pratique, cette concentration peut être considérée comme la limite inférieure du dosage. Pour un volume d'échantillon de 200 µl, la limite inférieure de dosage de MDA et/ou de 4-hydroxy-2-énaldéhydes dans l'échantillon correspond donc à une concentration 2,5 µmolaire.

Précision :

Lorsque six mesures sont effectuées le même jour sur quatre concentrations de standard comprises dans l'intervalle 0 à 20 µmolaire (dilutions extemporanées d'une solution-mère 10 mmolaire d'acétal standard conservée à +8°C), les coefficients de variation calculés sont tous inférieurs à 5 %.

Reproductibilité :

Lorsque l'expérience précédente est répétée à dix jours d'intervalle en utilisant la même solution-mère de standard conservée à +8°C, les nouveaux coefficients de variation, calculés à partir des deux séries de mesures, restent inférieurs à 5 %.

6. Interférences possibles.

L'héparinate de lithium ou de sodium entraîne une augmentation artéfactuelle des intensités d'absorbance à 586 nm.

Lors de l'incubation à 45°C, il se développe une coloration rose d'intensité variable, due à la formation de composés dont la longueur d'onde d'absorbance maximale est égale à 505 nm. L'absorption à 505 nm n'interfère généralement pas avec l'absorption du chromophore à 586 nm.

Remarques :

les échantillons peuvent être dilués notamment dans les milieux suivants : $H_2O$; tampon Tris-HCl 20 mmolaire, pH 7,4; tampon Tris-HCl 50 mmolaire, pH 7,6, en présence ou en l'absence de 0,1 % de Lubro®.

Toute modification des paramètres de dosage tels que température, tampon, pH, volume prélevé, est susceptible d'entraîner des modifications.

**EXEMPLES DE DOSAGES SUR ECHANTILLONS BIOLOGIOUES**

ENALDEHYDES.

1. Plasma.

On prélève 3 ml de sang dans un tube contenant 48 µl d'EDTA tripotassique 0,17 molaire. On centrifuge à 1700 x g et 4°C pendant 10 min, puis on sépare le plasma du culot érythrocytaire.
Le dosage doit être effectué dans l'heure qui suit, le plasma ayant été maintenu entre 0 et +4°C.
On effectue le dosage sur 200 µl de plasma, dans les conditions indiquées plus haut. Après 40 min d'incubation à 45°C en présence du réactif R2, on refroidit dans de la glace, centrifuge 10 min à 2500 x g et 4°C, et on mesure l'absorbance (A) du surnageant à 586 nm.
Les essais témoins ([aldéhyde] = 0) peuvent être effectués en remplaçant le plasma par de l'eau. Leur moyenne donne la valeur $A_0$.
La concentration totale en MDA et énaldéhydes, tels que définis précédemment, est déterminée à l'aide de ces mesures, comme expliqué plus haut.

2. Homogénat tissulaire.

Exemple : homogénat de cerveau de rat.

L'homogénat peut être réalisé au 1/10 (g/ml) dans du tampon Tris-HCl 20 mmolaire, pH 7,4.
Le dosage est effectué sur l'homogénat total ou sur un surnageant de centrifugation basse vitesse (1700 x g). On effectue le dosage comme décrit pour le plasma, avec une prise d'essai de 200 µl.
Pour un homogénat très peroxydé, on diminue la prise d'essai et on la complète à 200 µl avec le tampon précité.

DOSAGE SPECIFIOUE DU MDA.

Si, dans la procédure décrite ci-dessus on remplace l'acide méthanesulfonique (réactif R2) par un même volume d'acide chlorhydrique à 37 %, le plateau de la réaction est atteint au bout de 60 min et on obtient une mesure spécifique du MDA à 586 nm.
La figure 4 fournit un exemple de droite d'étalonnage obtenue dans ces conditions et montre que la réactivité du 4-HNE est pratiquement négligeable dans ces conditions.
Plus précisément, la figure 4 représente la droite d'étalonnage obtenue avec le tétraméthoxypropane (TMP), d'une part et avec le diacétal (diéthylacétal) du 4-hydroxynonénal [4-HNE (diacétal)], d'autre part après incubation en présence de HCl à 15 % (v/v), à 45°C, pendant 60 minutes.
La combinaison des procédures décrites ci-dessus (deux prises d'essai et deux dosages distincts) permet de calculer les concentrations des énaldéhydes, tels que définis précédemment, d'une part et du MDA, d'autre part.
Le coefficient d'extinction molaire apparent (E) obtenu à partir du MDA est le même dans les deux procédures.

**REFERENCES**

1. ESTERBAUER H. et CHEESEMAN K.H. (1990), Determination of aldehydic lipid peroxidation products: malonaldehyde and 4-hydroxynonenal; Meth. Enzymol., 186, 407-421.
2. DRAPER H.H. et HADLEY M. (1990), Malondialdehyde determination as index of lipid peroxidation; Meth. Enzymol., 186, 421-431.
3. FRANKEL E.N. et NEFF W.E. (1983), Formation of malonaldehyde from lipid oxidafion products; Biochim. Biophys. Acta, 754, 264-270.
4. HECKER M. et coll., (1987), Products, kinetics and substrate specificity of homogeneous thromboxane synthetase from human platelets: development of a novel enzyme assay, Arch. Biochem. Biophys., 254, 124.
5. JANERO D.R. (1990), Malondialdehyde and thiobarbituric acid-reactivity as diagnostic indices of lipid peroxidation and peroxidative tissue injury; Free Rad. Biol. Med., 9, 515-540.
6. SAWICKI E. et coll. (1963), Comparison of spectrophotometric and spectrofluorometric methods for the determination of malonaldehyde; Anal. Chem., 35, 199-205;
7. H. SCHERZ et coll., Mikrochim. Acta, 1967, 915.
8. KNIGHT J.A. et coll. (1988), Specificity of the thiobarbituric acid reaction: its use in studies of lipid peroxidation; Clin. Chem., 34, 2433-2438.

9. KOSUGI H. et KIKUGAWA K. (1986), Reaction of thiobarbituric acid with saturated aldehydes; Lipids, 21, 537-542.

10. KOSUGI H. et KIKUGAWA K. (1989), Potential thiobarbituric acid-reactive substances in peroxidized lipids; Free Rad. Biol. Med., 7, 205-207.

11. GUTTERIDGE J. MC. (1984), Reactivity, of hydroxyl and hydroxyl-like radicals discriminated by release of thiobarbituric acid-reactive material from deoxy sugars, nucleosides and benzoate; Biochem. J., 224, 761-767.

12. MATSUO T. et coll. (1987), Production of a malonaldehyde-like compound from the reaction of peroxide with sugar in acidic solution; Agric. Biol. Chem., 51, 2579-2580.

13. ESTERBAUER H. et coll. (1986), Studies on the mechanism of formation of 4-hydroxynonenal during microsomal lipid peroxidation; Biochim. Biophys. Acta, 876, 154-156.

14. SUNDBERG R.J.; The Chemistry of Indoles, 1970, Academic Press.

15. TAYLOR R.; Electrophilic Aromatic Substitution, 1990, J. Wiley & Sons, 187.

16. ARCADI A. et coll. (1989); Tetrahedron Letters, 30 (19), 2581-2584.

17. SAKAMOTO T. et coll. (1986); Heterocycles, 24 (7), 1845-1847.

18. UMEZAWA S. (1939); Bull. Chem. Soc. Japan, 14, 155.

19. B. SHIVARAMA HOLLA et SARVOTTAM Y. AMBEKAR (1978); Indian J. Chem., 16B, 240.

20. FENYES J.G.E (1974); J. Org. Chem. 27, 2614.

## Revendications

**1.** Procédé de dosage colorimétrique des énaldéhydes, ou du dialdéhyde malonique (MDA) seul, en tant qu'indices de peroxydation lipidique dans un milieu, notamment biologique, caractérisé en ce qu'il comprend essentiellement :

a) l'addition au dit milieu d'un réactif choisi parmi les composés de formule générale I ci-dessous et leurs sels d'addition éventuels à des bases organiques ou inorganiques, ou à des acides organiques ou inorganiques :

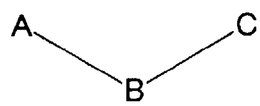

(I)

formule dans laquelle :

- A et C qui peuvent être identiques ou différents, représentent chacun H,

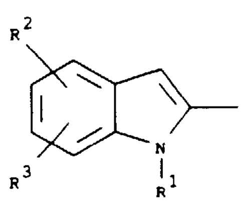

ou

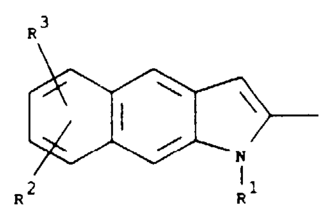

étant entendu que A et C ne peuvent représenter simultanément H, avec :

- $R^1$ = H; alkyle; aralkyle; aralkyle substitué sur le noyau aryle; alkylsulfonate, $Y^+$; alkylphosphonate, $Y^+$; ou alkylcarboxylate, $Y^+$;
- $R^2$ = H; $-OR^4$; F; Cl; Br; I; $-NO_2$; $-SO_3^-$ $Y^+$; -CN; $-COOR^4$; ou $-CONR^5R^6$;
- $R^3$ = H; $-OR^4$; $-NR^5R^6$; $-SR^4$: F; Cl: Br: I; $-NO_2$; $-SO_3^-$ $Y^+$; -CN; - $COR^5$; $-COOR^4$; ou $-CONR^5R^6$;
  - $R^4$ = H; alkyle; aralkyle; ou aralkyle substitué sur le noyau aryle;
  - $R^5$ = H; alkyle; aralkyle; ou aralkyle substitué sur le noyau aryle;
  - $R^6$ = H; aryle; aralkyle: ou aralkyle substitué sur le noyau aryle;
  - $Y^+$ = cation d'une base organique ou inorganique;
- B =

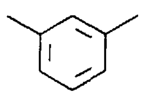 , 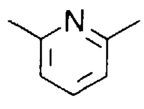 , 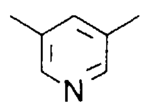 , 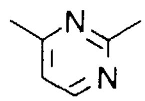 ,

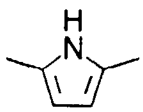 , 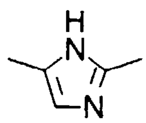 , 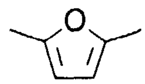 ,

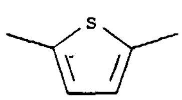 ou 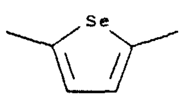

étant entendu que :

- alkyle représente un groupement linéaire ou ramifié comprenant 1 à 6 C;
- aralkyle substitué sur le noyau aryle signifie que celui-ci est substitué par un ou plusieurs groupements, identiques ou différents, choisis parmi alkyle (1 à 6 C), alcoxy (1 à 6 C), hydroxy, amino et carboxyle;

b) l'acidification du milieu ainsi additionné, soit par un acide choisi dans le groupe constitué par les acides carboxyliques forts, les acides sulfoniques forts et l'acide perchlorique, soit par l'acide chlorhydrique, l'acide bromhydrique ou l'acide sulfurique;
c) l'incubation du milieu ainsi acidifié à une température comprise entre 25 et 60°C, pendant une durée suffisante pour obtenir une coloration stable due à la formation d'un produit d'addition chromophorique du réactif avec tous les énaldéhydes, tels que définis précédemment, présents dans le milieu ou avec le dialdéhyde malonique seul, selon la nature de l'acide utilisé;
d) la mesure de l'absorbance du milieu coloré à la longueur d'onde d'absorption qui est spécifique du produit d'addition chromophorique du réactif utilisé, et son utilisation pour déterminer la concentration en énaldéhydes, tels que définis précédemment, ou en dialdéhyde malonique seul, selon le cas.

**2.** Procédé selon la revendication 1 pour le dosage colorimétrique des énaldéhydes, tels que définis précédemment caractérisé en ce que l'acidification est effectuée au moyen d'un acide carboxylique fort, d'un acide sulfonique fort ou d'acide perchlorique.

**3.** Procédé selon la revendication 1 pour le dosage colorimétrique du MDA seul, caractérisé en ce que l'acidification est effectuée au moyen d'acide chlorhydrique, bromhydrique ou sulfurique.

**4.** Procédé selon l'une quelconque des revendications 1 à 2, caractérisé en ce que le milieu réactionnel final contient un sel métallique, de préférence un sel de fer ferrique.

**5.** Procédé selon l'une quelconque des revendications 1 à 4, caractérisé en ce que la concentration en énaldéhydes, tels que définis précédemment, ou en dialdéhyde malonique seul, selon le cas, est déterminée à l'aide d'une courbe d'étalonnage établie au moyen d'une solution standard d'énaldéhyde, dans les mêmes conditions réactionnelles que le dosage à effectuer, en portant, en fonction de la concentration connue en énaldéhyde, la différence de l'absorbance A pour une concentration donnée et de l'absorbance $A_0$ d'un témoin ne contenant pas d'aldéhyde, ces absorbances étant lues à la longueur d'onde d'absorption maximale caractéristique du produit d'addition entre les énaldéhydes, tels que définis précédemment, et le composé de formule générale I utilisé.

**6.** Procédé selon la revendication 5, caractérisé en ce que la concentration en énaldéhyde(s) recherché(s) est déterminée à partir de la mesure de l'absorbance A de l'échantillon et de la mesure de l'absorbance A0 faite sur un témoin ne contenant pas d'aldéhyde, et du coefficient d'extinction molaire E qui correspond à la pente de la droite d'étalonnage, avec :

[concentration en énaldéhyde(s)] = $(A-A_0) \times K / E$,

où :

- la concentration en énaldéhyde(s) est exprimée en moles.$l^{-1}$;
- A et $A_0$ sont lues avec un trajet optique de 1 cm et sont exprimées en $cm^{-1}$;
- K est le facteur de dilution; et
- E est exprimé en 1.moles$^{-1}$.cm$^{-1}$.

**7.** Procédé selon l'une quelconque des revendications 1 à 6, caractérisé en ce que le composé de formule générale I est le 1-méthyl-2-phénylindole qui produit, avec les énaldéhydes, tels que définis précédemment, un chromophore stable dont la longueur d'onde d'absorption maximale est égale à 586 nm.

**8.** Nécessaire ou kit pour la mise en oeuvre du procédé selon l'une quelconque des revendications 1 à 6, caractérisé en ce qu'il comprend essentiellement un réactif de formule générale I, définie à la revendication 1.

**9.** Nécessaire ou kit selon la revendication 8, caractérisé en ce qu'il comprend en outre au moins un acide fort choisi dans le groupe constitué par les acides organiques forts comme l'acide trifluoroacétique, les acides sulfoniques forts comme l'acide méthanesulfonique ou l'acide trifluorométhanesulfonique, l'acide perchlorique, l'acide sulfurique, l'acide chlorhydrique et l'acide bromhydrique.

**10.** Nécessaire ou kit selon la revendication 8 ou 9, caractérisé en ce qu'il comprend en outre, en tant que référence ou échantillon standard, au moins un énaldéhyde, tel que défini précédemment, indice de la peroxydation lipidique ou un précurseur acido-labile d'un tel énaldéhyde, en solution aqueuse ou organique, à une concentration connue.

**11.** Composé de formule générale I:

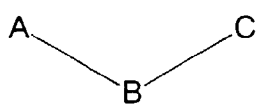

(I)

dans laquelle :

- A et C qui peuvent être identiques ou différents, ou représentent chacun H,

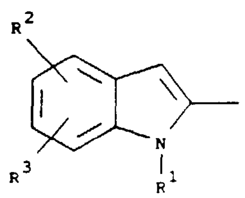

ou

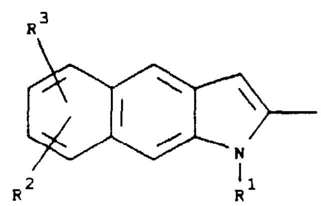

étant entendu que A et C ne peuvent représenter simultanément H, avec :

- $R^1$ = H; alkyle; aralkyle; aralkyle substitué sur le noyau aryle; alkylsulfonate, $Y^+$; alkylphosphonate, $Y^+$; ou alkylcarboxylate, $Y^+$;
- $R^2$ = H; $-OR^4$; F; Cl: Br; I: $-NO_2$; $-SO_3^-$ $Y^+$; -CN; $-COOR^4$; ou $-CONR^5R^6$;
- $R^3$ = H; $-OR^4$; $-NR^5R^6$; $-SR^4$; F; Cl; Br; I; $-NO_2$; $-SO_3^-$ $Y^+$; -CN; $-COR^5$;
  - $COOR^4$; ou $-CONR^5R^6$;
  - $R^4$ = H; alkyle; aralkyle; ou aralkyle substitué sur le noyau aryle;
  - $R^5$ = H; alkyle; aralkyle; ou aralkyle substitué sur le noyau aryle;

- $R^6$ = H; aryle; aralkyle; ou aralkyle substitué sur le noyau aryle;
- $Y^+$ = cation d'une base organique ou inorganique;

- B =

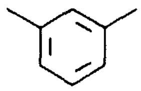 , 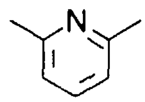 , 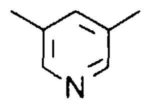 , 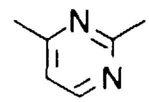 ,

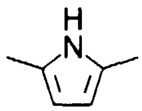 , 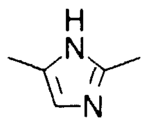 , 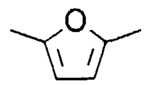 ,

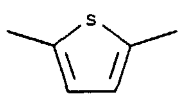 ou 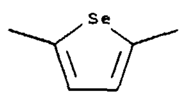

étant entendu que :

- alkyle représente un groupement linéaire ou ramifié comprenant 1 à 6 C;
- aralkyle substitué sur le noyau aryle signifie que celui-ci est substitué par un ou plusieurs groupements, identiques ou différents, choisis parmi $C_{1-6}$ alkyle, $C_{1-6}$ alcoxy, hydroxy, amino et carboxyle;
  étant entendu que A, B, C, ainsi que R1 à $R^6$ sont définis comme précédemment, étant entendu que
- lorsque A ou C représente H et l'autre substituant C ou A représente

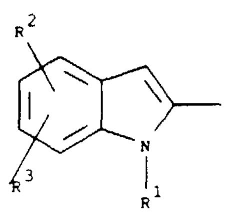

  $R^2$ et $R^3$ sont différents de H;-$OR^4$ ; et
- lorsque A ou C représente H, l'autre substituant C ou A représente

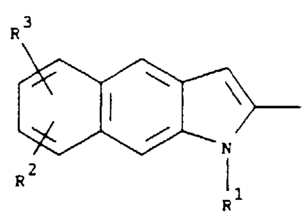

  et B représente

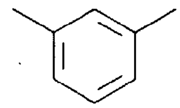

$R^1$, $R^2$ et $R^3$ ne représentent pas simultanément H.

## Patentansprüche

1. Verfahren zur kolorimetrischen Bestimmung von Enaldehyden, oder von Malondialdehyd (MDA) allein, als Lipidperoxidationsindices in einem insbesondere biologischen Medium, dadurch gekennzeichnet, daß es im wesentlichen umfaßt:

   a) Zusetzen, zum Medium, eines Reagenz, ausgewählt aus den Verbindungen der nachstehenden allgemeinen Formel (I) und ihren gegebenenfalls vorliegenden Additionssalzen mit organischen oder anorganischen Basen, oder mit organischen oder anorganischen Säuren:

   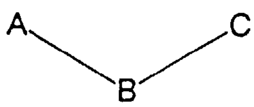

   (I),

   in welcher Formel:

   - A und C, die identisch oder verschieden sein können, jeweils H,

     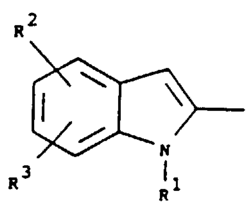

     oder

     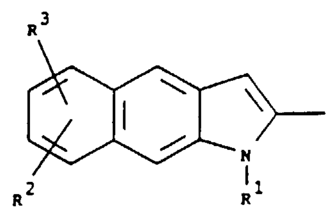

     darstellen, mit der Maßgabe, daß A und C nicht gleichzeitig H bedeuten können, wobei:
   - $R^1$ = H; Alkyl; Aralkyl; Aralkyl substituiert am Aryl-Kern; Alkylsulfonat, $Y^+$; Alkylphosphonat, $Y^+$; oder Alkylcarboxylat, $Y^+$;
   - $R^2$ = H; $-OR^4$; F; Cl; Br; I; $-NO_2$; $-SO_3^-Y^+$; -CN; $-COOR^4$; oder $-CONR^5R^6$;
   - $R^3$ = H; $-OR^4$; $-NR^5R^6$; $-SR^4$; F; Cl; Br; I; $-NO_2$; $-SO_3^-Y^+$; -CN; $-COR^5$; $-COOR^4$; oder $-CONR^5R^6$;
     - $R^4$ = H; Alkyl; Aralkyl; oder Aralkyl substituiert am Aryl-Kern;
     - $R^5$ = H; Alkyl; Aralkyl; oder Aralkyl substituiert am Aryl-Kern;
     - $R^6$ = H; Aryl; Aralkyl; oder Aralkyl substituiert am Aryl-Kern;
     - $Y^+$ = Kation einer organischen oder anorganischen Base;
   - B =

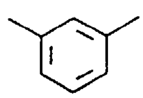 , 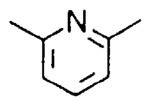 , 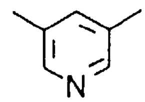 , 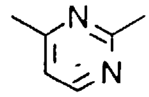 ,

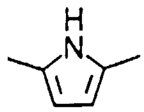 , 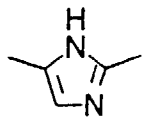 , 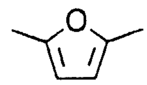 ,

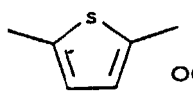 oder 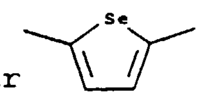

mit der Maßgabe, daß:

- Alkyl eine lineare oder verzweigte Gruppe mit 1 bis 6 C ist;
- Aralkyl substituiert am Aryl-Kern bedeutet, daß dieses substituiert ist durch eine oder mehrere, identische oder verschiedene Gruppen, ausgewählt aus Alkyl (1 bis 6 C), Alkoxy (1 bis 6 C), Hydroxy, Amino und Carboxyl;

b) Ansäuern des so ergänzten Mediums entweder mit einer Säure, ausgewählt aus der Gruppe bestehend aus starken Carbonsäuren, starken Sulfonsäuren und Perchlorsäure, oder mit Salzsäure, Bromwasserstoffsäure oder Schwefelsäure;
c) Inkubieren des so angesäuerten Mediums bei einer Temperatur zwischen 25 und 60°C während einer ausreichenden Dauer, um eine stabile Färbung zu erhalten, die auf die Bildung eines chromophoren Additionsprodukts des Reagenz mit allen im Medium vorliegenden Enaldehyden, wie vorstehend definiert, oder mit Malondialdeyhd allein in Abhängigkeit von der Beschaffenheit der verwendeten Säure zurückzuführen ist;
d) Messen des Absorptionsvermögens des gefärbten Mediums bei der Absorptionswellenlänge, die für das chromophore Additionsprodukt des verwendeten Reagenz spezifisch ist, und seine Verwendung zur Bestimmung der Konzentration je nach Fall von Enaldehyden, wie vorstehend definiert, oder von Malondialdehyd allein.

**2.** Verfahren nach Anspruch 1 zur kolorimetrischen Bestimmung von Enaldehyden, wie vorstehend definiert, dadurch gekennzeichnet, daß das Ansäuern mit einer starken Carbonsäure, einer starken Sulfonsäure oder Perchlorsäure durchgeführt wird.

**3.** Verfahren nach Anspruch 1 zur kolorimetrischen Bestimmung von MDA allein, dadurch gekennzeichnet, daß das Ansäuern mit Salz-, Bromwasserstoff- oder Schwefelsäure durchgeführt wird.

**4.** Verfahren nach einem der Ansprüche 1 oder 2, dadurch gekennzeichnet, daß das End-Reaktionsmedium ein Metallsalz, vorzugsweise ein Eisen(III)-salz enthält.

**5.** Verfahren nach einem der Ansprüche 1 bis 4, dadurch gekennzeichnet, daß die Konzentration je nach Fall von Enaldehyden, wie vorstehend definiert, oder von Malondialdehyd allein mit Hilfe einer Eichkurve bestimmt wird, die mit einer Standard-Enaldehyd-Lösung unter denselben Reaktionsbedingungen wie die durchzuführende Bestimmung erstellt wird, indem, als Funktion der bekannten Enaldehyd-Konzentration, die Differenz des Absorptionsvermögens A für eine gegebenen Konzentration und des Absorptionsvermögens $A_0$ einer keinen Aldehyd enthaltenden Kontrolle aufgetragen wird, wobei diese Absorptionsvermögen bei der maximalen Absorptionswellenlänge abgelesen werden, die für das Additionsprodukt zwischen den Enaldehyden, wie vorstehend definiert, und der verwendeten Verbindung der allgemeinen Formel (I) charakteristisch ist.

**6.** Verfahren nach Anspruch 5, dadurch gekennzeichnet, daß die Konzentration von gesuchtem Enaldehyd bzw. Enaldehyden bestimmt wird durch die Messung des Absorptionsvermögens A der Probe und die Messung des

Absorptionsvermögens $A_0$ einer keinen Aldehyd enthaltenden Kontrolle, und den molaren Extinktionskoeffizienten E, welcher der Steigung der Meßgeraden entspricht, wobei:

$$[\text{Konzentration von Enaldehyd(en)}]=(A-A_0) \times K/E,$$

worin:

- die Konzentration von Enaldehyd(en) in mol x $1^{-1}$ ausgedrückt wird;
- A und $A_0$ mit einem Lichtweg von 1 cm abgelesen werden und in $cm^{-1}$ ausgedrückt werden;
- K der Verdünnungsfaktor ist; und
- E in 1 x $mol^{-1}$ x $cm^{-1}$ ausgedrückt wird.

7. Verfahren nach einem der Ansprüche 1 bis 6, dadurch gekennzeichnet, daß die Verbindung der allgemeinen Formel (I) 1-Methyl-2-phenylindol ist, die mit den Enaldehyden, wie vorstehend definiert, einen stabilen Chromophoren erzeugt, dessen maximale Absorptionswellenlänge 586 nm beträgt.

8. Testsatz oder Kit zur Durchführung des Verfahrens nach einem der Ansprüche 1 bis 6, dadurch gekennzeichnet, daß er im wesentlichen ein Reagenz der in Anspruch 1 definierten allgemeinen Formel (I) umfaßt.

9. Testsatz oder Kit nach Anspruch 8, dadurch gekennzeichnet, daß er außerdem zumindest eine starke Säure umfaßt, ausgewählt aus der Gruppe bestehend aus starken organischen Säuren, wie Trifluoressigsäure, starken Sulfonsäuren, wie Methansulfonsäure oder Trifluormethansulfonsäure, Perchlorsäure, Schwefelsäure, Salzsäure und Bromwasserstoffsäure.

10. Testsatz oder Kit nach Anspruch 8 oder 9, dadurch gekennzeichnet, daß er außerdem, als Referenz oder Standard-Probe, zumindest einen Enaldehyd, wie vorstehend defniert, als Lipidperoxidationsindex oder einen säurelabilen Vorläufer eines derartigen Enaldehyds in wässeriger oder organischer Lösung bei einer bekannten Konzentration enthält.

11. Verbindung der allgemeinen Formel (I):

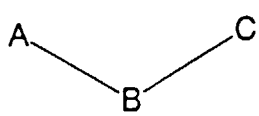

(I),

in welcher:

- A und C, die identisch oder verschieden sein können, jeweils H,

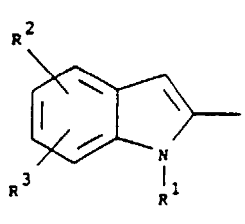

oder

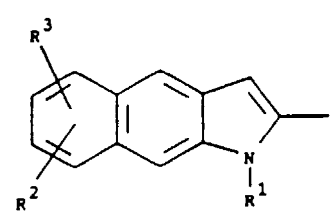

darstellen, mit der Maßgabe, daß A und C nicht gleichzeitig H bedeuten können, wobei:

- $R^1$ = H; Alkyl; Aralkyl; Aralkyl substituiert am Aryl-Kern; Alkylsulfonat, $Y^+$; Alkylphosphonat, $Y^+$; oder Alkylcarboxylat, $Y^+$;
- $R^2$ = H; $-OR^4$; F; Cl; Br; I; $-NO_2$;$-SO_3^-Y^+$;-CN;$-COOR^4$; oder $-CONR^5R^6$;
- $R^3$ = H; $-OR^4$; $-NR^5R^6$; $-SR^4$; F; Cl; Br; I; $-NO_2$; $-SO_3^-Y^+$; -CN; $-COR^5$; $-COOR^4$; oder $-CONR^5R^6$;

- $R^4$ = H; Alkyl; Aralkyl; oder Aralkyl substituiert am Aryl-Kern;
- $R^5$ = H; Alkyl; Aralkyl; oder Aralkyl substituiert am Aryl-Kern;
- $R^6$ = H; Aryl; Aralkyl; oder Aralkyl substituiert am Aryl-Kern;
- $Y^+$ = Kation einer organischen oder anorganischen Base;

- B =

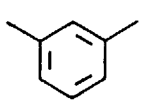 , 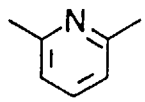 ., 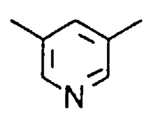 , 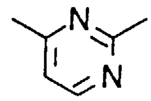 ,

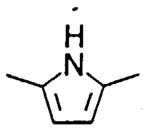 , 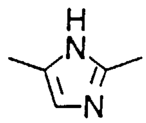 , 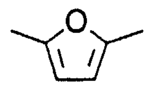 ,

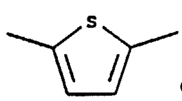 oder 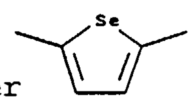

mit der Maßgabe, daß:

- Alkyl eine lineare oder verzweigte Gruppe mit 1 bis 6 C ist;
- Aralkyl substituiert am Aryl-Kern bedeutet, daß dieses substituiert ist durch eine oder mehrere, identische oder verschiedene Gruppen, ausgewählt aus $C_{1-6}$-Alkyl, $C_{1-6}$-Alkoxy, Hydroxy, Amino und Carboxyl;

mit der Maßgabe, daß A, B, C sowie $R^1$ bis $R^6$ wie vorstehend definiert sind,
mit der Maßgabe, daß

- wenn A oder C die Bedeutung H hat, und der andere Substituent C oder A

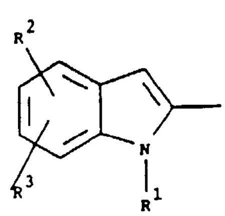

bedeutet, $R^2$ und $R^3$ verschieden sind von H; -$OR^4$; und
- wenn A oder C die Bedeutung H hat, der andere Substituent C oder A

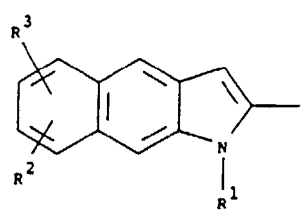

bedeutet, und B

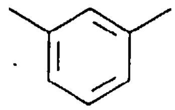

darstellt, $R^1$, $R^2$ und $R^3$ nicht gleichzeitig H sein können.

**Claims**

**1.** Method for the colorimetric analysis of enaldehydes, or malonic dialdehyde (MDA) by itself, as indexes of lipid peroxidation in a medium, especially a biological medium, characterized in that it comprises essentially:

a) adding to said medium a reagent selected from the compounds of general formula I below and their possible addition salts with organic or inorganic bases or with organic or inorganic acids:

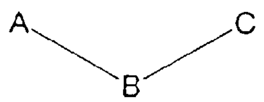

(I)

in which formula:

- A and C, which can be identical or different, are each H,

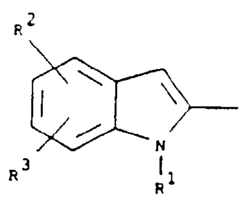

or

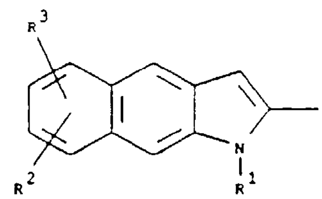

it being understood that A and C cannot simultaneously be H, where:
- $R^1$ = H; alkyl; aralkyl; aralkyl substituted on the aryl ring; alkylsulfonate.$Y^+$; alkylphosphonate.$Y^+$; or alkylcarboxylate. $Y^+$;
- $R^2$ = H; $-OR^4$; F; Cl; Br; I;$-NO_2$; $-SO_3^-Y^+$; -CN; $-COOR^4$; or $-CONR^5R^6$;
- $R^3$ = H; $-OR^4$; $-NR^5R^6$; $-SR^4$; F; Cl; Br; I; $-NO_2$; $-SO_3^-Y^+$; -CN; $-COR^5$; $-COOR^4$; or $-CONR^5R^6$;
  - $R^4$ = H; alkyl; aralkyl; or aralkyl substituted on the aryl ring;
  - $R^5$ = H; alkyl; aralkyl; or aralkyl substituted on the aryl ring;
  - $R^6$ = H; aryl; aralkyl; or aralkyl substituted on the aryl ring; and
  - $Y^+$ = cation of an organic or inorganic base; and
- B =

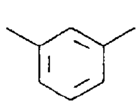 , 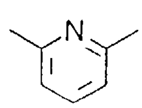 , 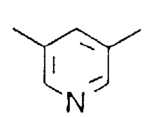 , 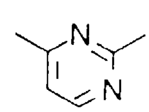 ,

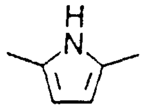 , 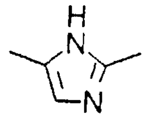 , 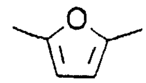 ,

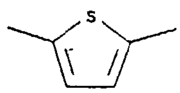 or 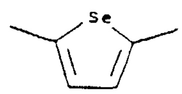

it being understood that:

- alkyl is a linear or branched group comprising 1 to 6 C; and
- aralkyl substituted on the aryl ring denotes that said ring is substituted by one or more identical or different groups selected from alkyl (1 to 6 C), alkoxy (1 to 6 C), hydroxyl, amino and carboxyl;

b) acidifying the medium to which this reagent has been added, either with an acid selected from the group comprising strong carboxylic acids, strong sulfonic acids and perchloric acid, or with hydrochloric acid, hydrobromic acid or sulfuric acid;
c) incubating the medium acidified in this way, at a temperature between 25 and 60°C, for a sufficient time to obtain a stable coloration due to the formation of a chromophoric addition product of the reagent with all the enaldehydes, as defined above, present in the medium, or with malonic dialdehyde by itself, depending on the nature of the acid used; and
d) measuring the absorbance of the coloured medium at the absorption wavelength which is specific for the chromophoric addition product of the reagent used, and using this to determine the concentration of enaldehydes, as defined above, or of malonic dialdehyde by itself, depending on the particular case.

**2.** Method according to Claim 1 for the colorimetric analysis of enaldehydes, as defined above, characterized in that the acidification is effected by means of a strong carboxylic acid, a strong sulfonic acid or perchloric acid.

**3.** Method according to Claim 1 for the colorimetric analysis of MDA by itself, characterized in that the acidification is effected by means of hydrochloric, hydrobromic or sulfuric acid.

**4.** Method according to either one of Claims 1 and 2, characterized in that the final reaction medium contains a metal salt, preferably a ferric iron salt.

**5.** Method according to any one of Claims 1 to 4, characterized in that the concentration of enaldehydes, as defined above, or of malonic dialdehyde by itself, depending on the particular case, is determined with the aid of a calibration curve established by means of a standard solution of enaldehyde under the same reaction conditions as the analysis to be performed, the difference in the absorbance A for a given concentration and the absorbance $A_0$ of a reference not containing aldehyde being plotted as a function of the known concentration of enaldehyde, these absorbances being read at the maximum absorption wavelength characteristic of the addition product between the enaldehydes, as defined above, and the compound of general formula I used.

**6.** Method according to Claim 5, characterized in that the concentration of enaldehyde(s) to be analyzed is determined from the measurement of the absorbance A of the sample and the absorbance $A_0$ of a reference not containing aldehyde, and from the measurement of the molar extinction coefficient E corresponding to the slope of the calibration line, where:

$$[\text{concentration of enaldehyde(s)}] = (A - A_0) \times K / E,$$

in which:

- the concentration of enaldehyde(s) is expressed in $mol.l^{-1}$ ;
- A and $A_0$ are read with an optical path length of 1 cm and are expressed in $cm^{-1}$;
- K is the dilution factor; and
- E is expressed in $1.mol^{-1}.cm^{-1}$.

**7.** Method according to any one of Claims 1 to 6, characterized in that the compound of general formula I is l-methyl-2-phenylindole, which produces, with enaldehydes, as defined above, a stable chromophore with a maximum absorption wavelength of 586 nm.

**8.** Kit for carrying out the method according to any one of Claims 1 to 6, characterized in that it comprises essentially a reagent of general formula I defined in Claim 1.

**9.** Kit according to Claim 8, characterized in that it also comprises at least one strong acid selected from the group consisting of strong organic acids like trifluoroacetic acid, strong sulfonic acids like methanesulfonic acid or trifluoromethanesulfonic acid, perchloric acid, sulfuric acid, hydrochloric acid and hydrobromic acid.

**10.** Kit according to Claim 8 or 9, characterized in that it also comprises, as a reference or standard sample, at least one enaldehyde, as defined above, as an index of lipid peroxidation, or an acid-labile precursor of said enaldehyde, at a known concentration in aqueous or organic solution.

**11.** Compound of general formula I:

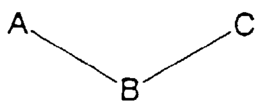

(I)

in which:

- A and C, which can be identical or different, are each H,

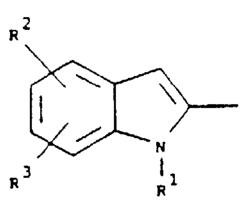

or

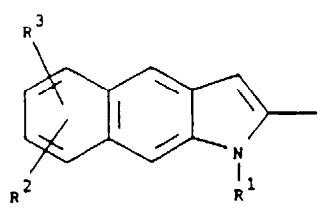

it being understood that A and C cannot simultaneously be H, where:
- $R^1$ = H; alkyl; aralkyl; aralkyl substituted on the aryl ring; alkylsulfonate.$Y^+$; alkylphosphonate.$Y^+$; or alkylcarboxylate.$Y^+$;
- $R^2$ = H; $-OR^4$; F; Cl; Br; I; $-NO_2$; $-SO_3^-Y^+$; -CN; $-COOR^4$; or $-CONR^5R^6$;
- $R^3$ = H; $-OR^4$; $-NR^5R^6$; $-SR^4$; F; Cl; Br; I; $-NO_2$; $-SO_3^-Y^+$; -CN; $-COR^5$; $-COOR^4$; or $-CONR^5R^6$;

  - $R^4$ = H; alkyl; aralkyl; or aralkyl substituted on the aryl ring;
  - $R^5$ = H; alkyl; aralkyl; or aralkyl substituted on the aryl ring;
  - $R^6$ = H; aryl; aralkyl; or aralkyl substituted on the aryl ring; and
  - $Y^+$ = cation of an organic or inorganic base; and

- B =

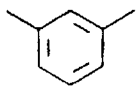 , 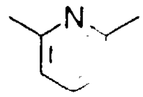 , 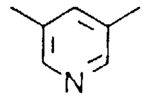 , 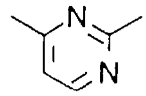 ,

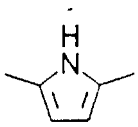 , 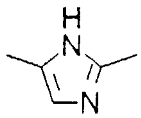 , 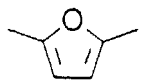 ,

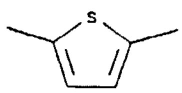 or 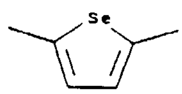

it being understood that:

- alkyl is a linear or branched group comprising 1 to 6 C; and
- aralkyl substituted on the aryl ring denotes that said ring is substituted by one or more identical or different groups selected from $C_{1-6}$-alkyl, $C_{1-6}$-alkoxy, hydroxyl, amino and carboxyl;

it being understood that A, B, C and $R^1$ to $R^6$ are as defined above, and it being understood that:

- if A or C is H and the other substituent C or A is

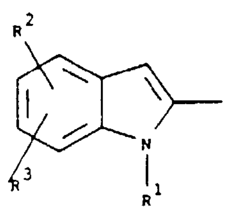

  $R^2$ and $R^3$ are other than H or $-OR^4$; and
- if A or C is H, the other substituent C or A is

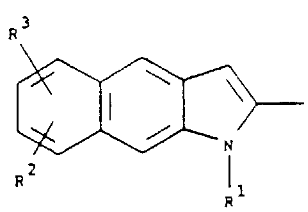

  and B is

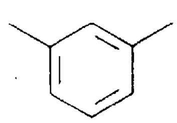

$R^1$, $R^2$ and $R^3$ are not simultaneously H.

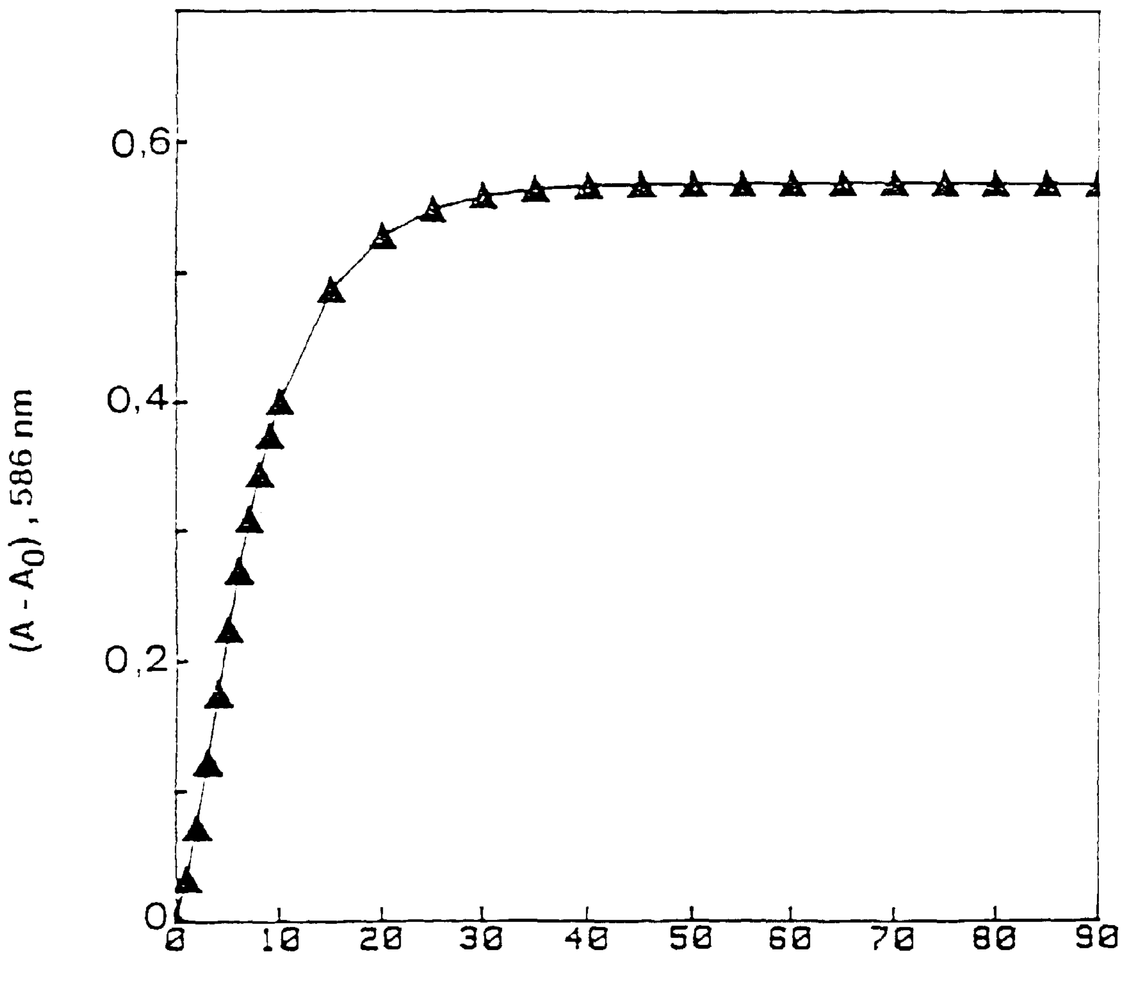


Cinétique de formation de l'adduit du tétraméthoxypropane (TMP) avec le 1-méthyl-2-phénylindole (MPI)

Fig. 1

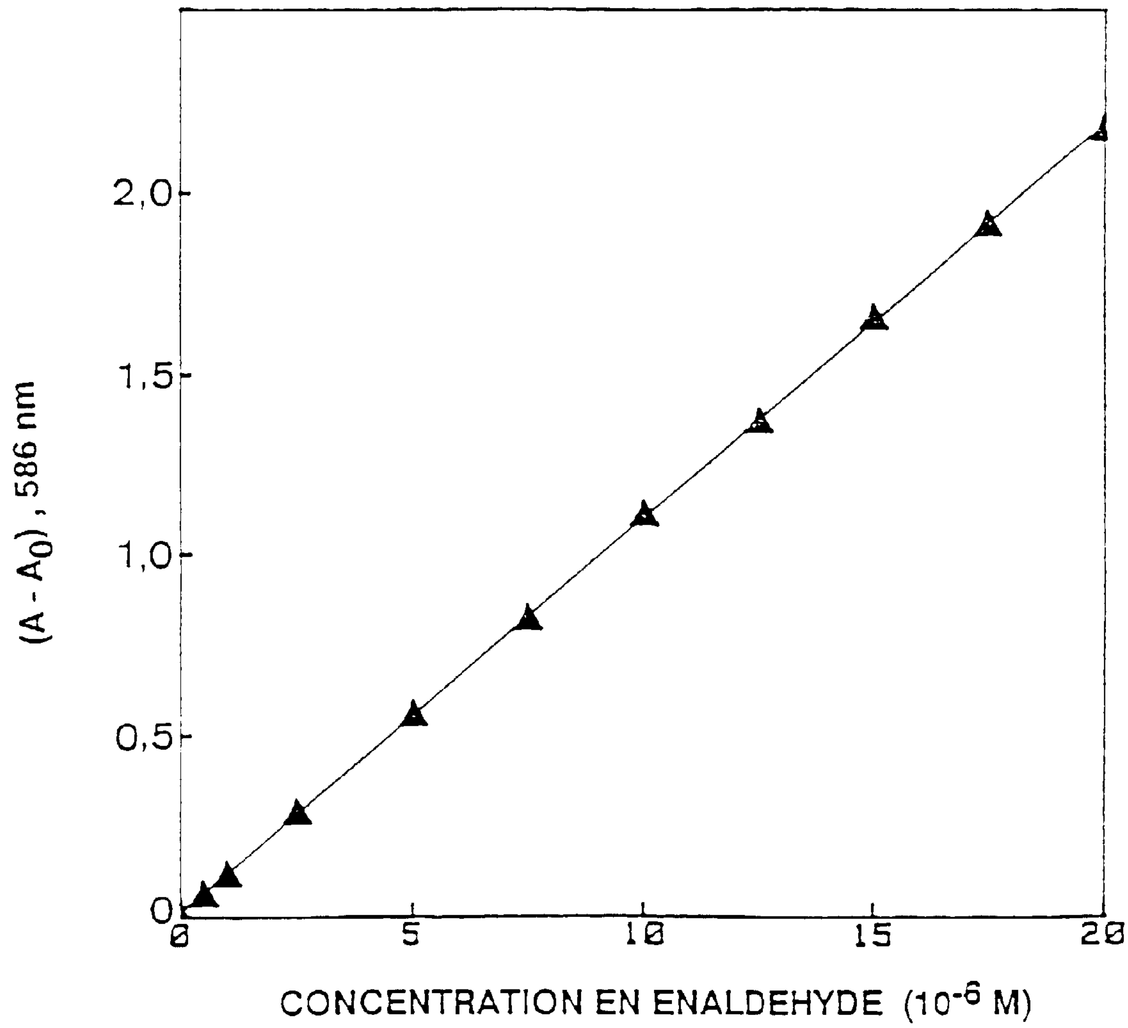


Courbe d'étalonnage avec le tétraméthoxypropane

Fig. 2

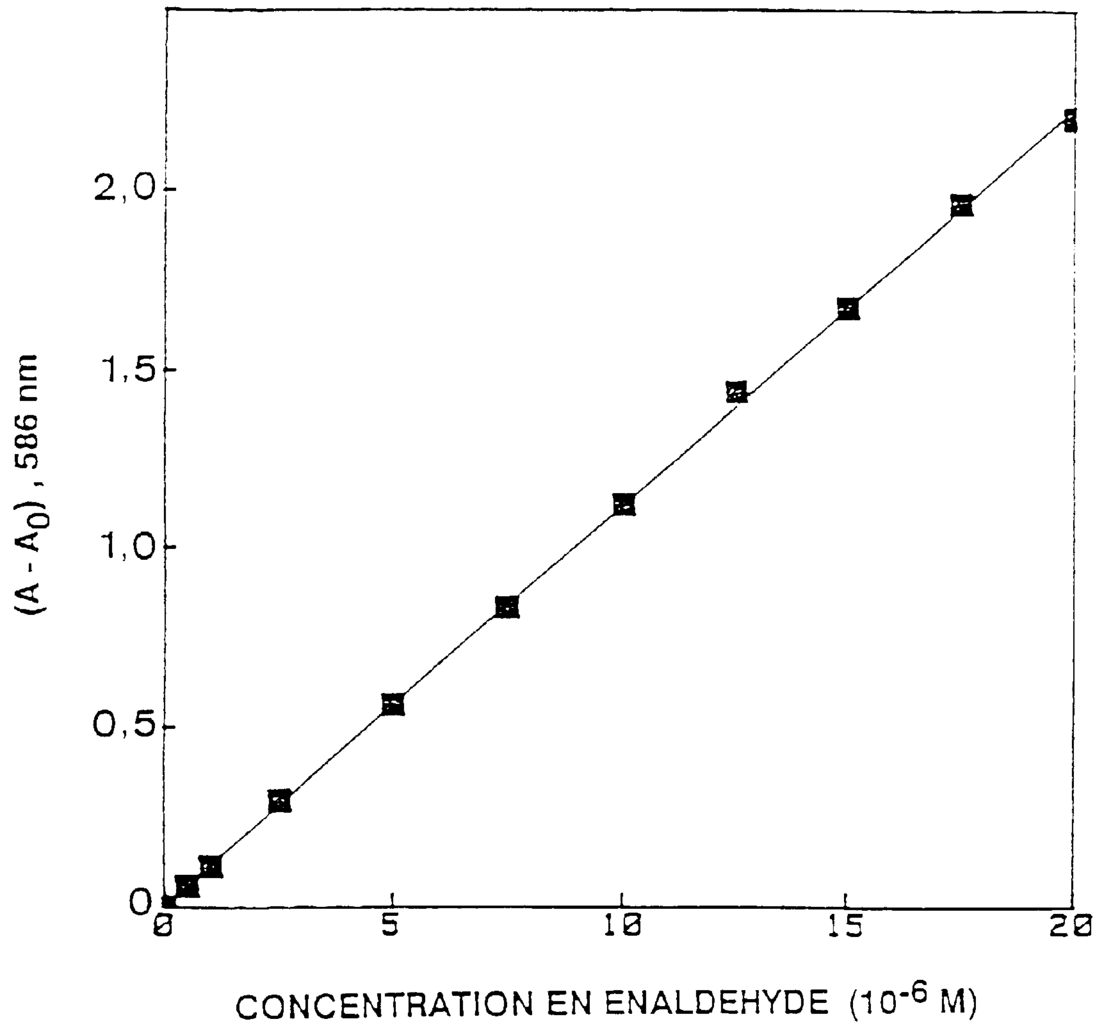


Courbe d'étalonnage avec le diéthylacétal du 4-hydroxynonénal

Fig. 3

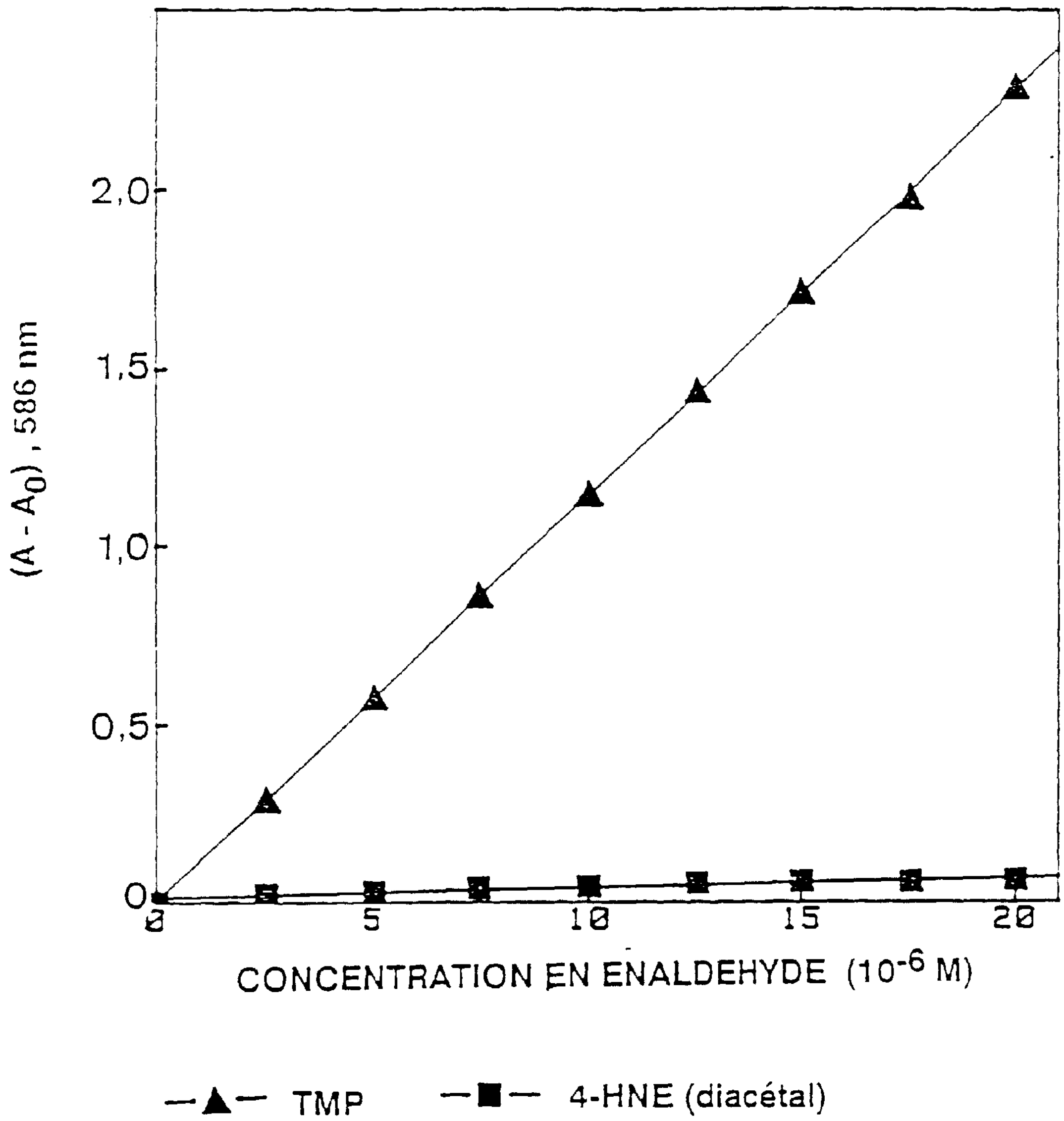


Courbes d'étalonnage en présence d'acide chlorhydrique

Fig. 4